# EUROPÄISCHE PATENTSCHRIFT

(11) **EP 1 335 936 B1**
(45) Veröffentlichungstag und Bekanntmachung des Hinweises auf die Patenterteilung: **27.09.2006**
(21) Anmeldenummer: 01949334.5
(22) Anmeldetag: 10.05.2001
(51) Int. Cl.: C07K 14/40, C07K 16/14, C12Q 1/68, G01N 33/53

(54) **HYPHENSPEZIFISCHE FAKTOREN AUS CANDIDA ALBICANS**
HYPHA-SPECIFIC FACTORS FROM CANDIDA ALBICANS
FACTEURS SPECIFIQUES AUX FILAMENTS MYCELLIENS EXTRAITS DE CANDIDA ALBICANS

(30) Priorität: 11.05.2000 DE 10023130
(43) Veröffentlichungstag der Anmeldung: 20.08.2003
(73) Patentinhaber: Fraunhofer-Gesellschaft zur Förderung der angewandten Forschung e.V., 80686 München (DE)
(72) Erfinder: RUPP, Steffen, 70195 Stuttgart (DE); JOHANNES, Franz-Josef, 71229 Leonberg (DE); SOHN, Kai, 73527 Schwäbisch-Gmünd (DE)
(86) Internationale Anmeldenummer: PCT/EP2001/005363
(87) Internationale Veröffentlichungsnummer: WO 2001/085989

(56) Entgegenhaltungen:
- WO-A-97/31256
- WO-A-98/18927
- WO-A-99/39210
- WO-A-99/40434
- US-A- 5 776 694
- DATABASE EBI [Online] EMBL; 20. Juni 1997 (1997-06-20) CHOI, S.Y.: "Candida sp. HN95 CIP1-Gene" retrieved from EBI, accession no. Y13973 Database accession no. Y13973 XP002231416
- DATABASE TREMBL [Online] EBI; 1. Juli 1997 (1997-07-01) HONG, Y. ET AL.: "Cadmium induced CIP1 protein" retrieved from EMBL, accession no. P87221 Database accession no. P87221 XP002231417
- DATABASE EBI [Online] EMBL; 11. Mai 1994 (1994-05-11) RASMUSSEN, S. ET AL.: "S. cerevisiae chromosome XI readingframe ORF YKL067w" retrieved from EMBL, accession no. Z28067 Database accession no. Z28076 XP002231418
- DATABASE TREMBL [Online] EMBL; 1. Juni 1994 (1994-06-01) RASMUSSEN, S.W.: "Nucleoside Diphosphate Kinase (EC 2.7.4.6)" retrieved from EMBL, accession no. P36010 Database accession no. P36010 XP002231419
- DATABASE TREMBL [Online] EMBL; 14. April 1995 (1995-04-14) BUDDE, E. ET AL.: "Saccharomyces cereviseae, hydroperoxide resistance (HYR1) gene, complete cds" retrieved from EMBL, accession no. U22446 Database accession no. U22446 XP002231420
- DATABASE TREMBL [Online] EMBL; 1. Januar 1990 (1990-01-01) SCHWELBERGER, H.G. ET AL.: "Fructose-bisphosphate aldolase" retrieved from EMBL, accession no. P14540 Database accession no. P14540 XP002231421
- DATABASE EBI [Online] EMBL; 15. März 1991 (1991-03-15) SCHWELBERGER, H.G. ET AL.: "YEAST FBA1 gene for fructose-biphosphate aldolase" retrieved from EMBL, accession no. X15003 Database accession no. X15003 XP002231422
- PARAVICINI GERHARD ET AL: "The Candida albicans PKC1 gene encodes a protein kinase C homolog necessary for cellular integrity but not dimorphism." YEAST, Bd. 12, Nr. 8, 1996, Seiten 741-756, XP008013926 ISSN: 0749-503X

### SEQUENZPROTOKOLL

<110> Fraunhofer-Gesellschaft zur Förderung.....
<120> Hyphenspezifische Faktoren aus Candida albicans
<130> 23874
<140>
   <141>
<160> 18
<170> Patent In Ver. 2.1
<210> 1
   <211> 900
   <212> DNA
   <213> Candida albicans
<400> 1
   atgtctaaag tctcaattac tatcatcggt ttgaatggtt tcttaggtaa accagttctt 60
   gaagctatca attctggtat ttttgatgat aagatcaact tcccaatcaa ggcaattacc 120
   agaaaggaac cagaaactaa gaatgacaaa attgaatatg ttgtttctga aatcaatgaa 180
   gaatcaatta aatcaacttt gagccaaaaa ttatctggta ctgatgttat tattgaatta 240
   attggtccaa atccagaggc tttcgccaat atcgaaaatt tagttgatgc aattaaacca 300
   aaattattta tcccatcaca atttggtact gatattccta aagttgatga atatgctcca 360
   gggtttttag gaattaaaac tcaacattca gaaaatgtca gaaaatcagg agttaaagtt 420
   gttgatatta taacttcgtt atttgctgtt ccaggagctt ttctttatga atgggttggt 480
   tcaactggta ttaatgctga agacagaact gttaaactca ttggtgacat taatcaacaa 540
   tttgatattt ctaaattaga agatgttggt aaagctgtac tttctattgc tactaatcct 600
   aatccaagag aattaccaga taccattaga attggttctg atagaattac tgttaaagat 660
   gtaattgata gatactctaa agatcataat gttgaattga aaattgtttc agaacaatct 720
   gcagaagacg ccaagaaaga gtttactgaa tctttgaaag ttggttttga tggtgataaa 780
   ttcttatggt atttacaagt tattgctgct caaggtttag ataaaggttt actctccagt 840
   aaattggata atgaattggt taatccaggt gaatctttat ggaaatgggg caagtactaa 900
<210> 2
   <211> 900
   <212> DNA
   <213> Candida albicans
<400> 2
   atgtctaaag tctcaattac tatcatcggt ttgaatggtt tcttaggtaa accagttctt 60
   gaagctatca attctggtat ttttgacgat aaaatcaatt tcccaattaa agctattaca 120
   agaaaagaac cggaaactaa aaatgacaaa attgaatacg ttgtttctga aatcaatgaa 180
   gaatcaatta aatcaacctt gagccaaaaa ttatccggta ctgatgttat tattgaatta 240
   attggtccaa atccagaggc tttcgctaat atcgaaaaat taattgatgc aattaaacca 300
   aaattattca ttccatcaca atttggtact gatattccta aagttgatga atatgctcca 360
   gggtttttag gaatcaaaac tcaacattca gaaaatgtca gaaaattagg agttaaagtt 420
   gttgatatta taacttcgtt atttgctgtt ccaggagctt ttctttatga atgggttggt 480
   tcaactggta tcaatgctga tgacaaaact gttaaactta ttggtgacat taatcaacaa 540
   tttgatattt ctaaattaga agatgttggt aaagctgtac tttctattgc tactaatcct 600
   aatccaagag aattaccaga taccattaga attggttctg atagaattac tgtcaaagat 660
   gtcattgata gatattctaa agatcataat gttgaattga aagttgtttc tgaacaatct 720
   gcagaagatg ccaagaaaga gtttactgaa tctttgaaag ctggttttga tggtgagaaa 780
   ttcttatggt atttacaagt tattgctgct caaggtttag ataaaggttt actctccagt 840
   aaattggaca acgaattggt caacccaggt gagtctttat ggaaatgggg caagtactaa 900
<210> 3
   <211> 501
   <212> DNA
   <213> Candida albicans
<400> 3
   atggcctcct cagtaaagtt ggctacggca cttaaacaac gtgctatatt gacaaaagaa 60
   ttgtctgaat tagatgataa aatacaatct tcattgattc tgcaagttgg tatgaaaaaa 120
   atcaatgatc cagataaatt gtatttagat tatgttgcta aatctcaaga attggctaaa 180
   ttggtatcat caataaatta tactaataat ataactccaa ttgaacttga tttgacaatg 240
   ggaaagtatg ataatactat aaaaacaatt aatgatgcat taatttgtcg agaccgaata 300
   tttaaaaaat tacaatttgt gaaaaaaata tcaacagcag gtaaagaaca accattagat 360
   tccaaagatg aaattaaatt tgtatcattt attgatgttg ataaatatga tactttggcc 420
   caagaattaa atactcaatt tgagaatttg aatttgaaat tacaagaaat aaattggcaa 480
   gttgatcttg ttgagatata a                                           501
<210> 4
   <211> 486
   <212> DNA
   <213> Candida albicans
<400> 4
   atgaaattag ctgaagcatt aaatttaaaa aagaacttgg aaagagatgc tggtgaactt 60
   aaatcattaa ttcttaaatg ttgtcaagct caaactggcg aaaaccctcc atttgatcct 120
   aatgaattat ttgaacaata tgaagaaatt gataaattaa ttactgatat aactattaaa 180
   atacaacgaa ccaataatga aataaagttt gcctatgata atgataataa gtctaatgaa 240
   gaaccacttc gatcaatgac acaagctatt gctgatattg atgatttaga aagacaaatc 300
   aatgtgacag atgatataat tcataatggt attattacaa aactgtattc gaccaagaag 360
   attgctgatg tgtcacatgt tgacgtggtt gcatatgaca agacaagaaa gaaaatgaat 420
   gagagattag ataaattaaa acttcgtata cagtcggcaa attgggaatt tgatctaatt 480
   gattaa                                                            486
<210> 5
   <211> 299
   <212> PRT
   <213> Candida albicans
<400> 5
   Met Ser Lys Val Ser Ile Thr Ile Ile Gly Leu Asn Gly Phe Leu Gly
   1               5                   10                  15
   Lys Pro Val Leu Glu Ala Ile Asn Ser Gly Ile Phe Asp Asp Lys Ile
               20                  25                  30
   Asn Phe Pro Ile Lys Ala Ile Thr Arg Lys Glu Pro Glu Thr Lys Asn
           35                  40                  45
   Asp Lys Ile Glu Tyr Val Val Ser Glu Ile Asn Glu Glu Ser Ile Lys
       50                  55                  60
   Ser Thr Leu Ser Gln Lys Leu Ser Gly Thr Asp Val Ile Ile Glu Leu
   65                  70                  75                  80
   Ile Gly Pro Asn Pro Glu Ala Phe Ala Asn Ile Glu Asn Leu Val Asp
                   85                  90                  95
   Ala Ile Lys Pro Lys Leu Phe Ile Pro Ser Gln Phe Gly Thr Asp Ile
               100                 105                 110
   Pro Lys Val Asp Glu Tyr Ala Pro Gly Phe Leu Gly Ile Lys Thr Gln
           115                 120                 125
   His Ser Glu Asn Val Arg Lys Ser Gly Val Lys Val Val Asp Ile Ile
           130                 135                 140
   Thr Ser Leu Phe Ala Val Pro Gly Ala Phe Leu Tyr Glu Trp Val Gly
   145                 150                 155                 160
   Ser Thr Gly Ile Asn Ala Glu Asp Arg Thr Val Lys Leu Ile Gly Asp
                   165                 170                 175
   Ile Asn Gln Gln Phe Asp Ile Ser Lys Leu Glu Asp Val Gly Lys Ala
               180                 185                 190
   Val Leu Ser Ile Ala Thr Asn Pro Asn Pro Arg Glu Leu Pro Asp Thr
           195                 200                 205
   Ile Arg Ile Gly Ser Asp Arg Ile Thr Val Lys Asp Val Ile Asp Arg
       210                 215                 220
   Tyr Ser Lys Asp His Asn Val Glu Leu Lys Ile Val Ser Glu Gln Ser
   225                 230                 235                 240
   Ala Glu Asp Ala Lys Lys Glu Phe Thr Glu Ser Leu Lys Val Gly Phe
                   245                 250                 255
   Asp Gly Asp Lys Phe Leu Trp Tyr Leu Gln Val Ile Ala Ala Gln Gly
               260                 265                 270
   Leu Asp Lys Gly Leu Leu Ser Ser Lys Leu Asp Asn Glu Leu Val Asn
           275                 280                 285
   Pro Gly Glu Ser Leu Trp Lys Trp Gly Lys Tyr
       290                 295
<210> 6
   <211> 299
   <212> PRT
   <213> Candida albicans
<400> 6
   Met Ser Lys Val Ser Ile Thr Ile Ile Gly Leu Asn Gly Phe Leu Gly
    1               5                  10                  15
   Lys Pro Val Leu Glu Ala Ile Asn Ser Gly Ile Phe Asp Asp Lys Ile
               20                  25                  30
   Asn Phe Pro Ile Lys Ala Ile Thr Arg Lys Glu Pro Glu Thr Lys Asn
           35                  40                  45
   Asp Lys Ile Glu Tyr Val Val Ser Glu Ile Asn Glu Glu Ser Ile Lys
       50                  55                  60
   Ser Thr Leu Ser Gln Lys Leu Ser Gly Thr Asp Val Ile Ile Glu Leu
   65                  70                  75                  80
   Ile Gly Pro Asn Pro Glu Ala Phe Ala Asn Ile Glu Lys Leu Ile Asp
                   85                  90                  95
   Ala Ile Lys Pro Lys Leu Phe Ile Pro Ser Gln Phe Gly Thr Asp Ile
               100                 105                 110
   Pro Lys Val Asp Glu Tyr Ala Pro Gly Phe Leu Gly Ile Lys Thr Gln
           115                 120                 125
   His Ser Glu Asn Val Arg Lys Leu Gly Val Lys Val Val Asp Ile Ile
       130                 135                 140
   Thr Ser Leu Phe Ala Val Pro Gly Ala Phe Leu Tyr Glu Trp Val Gly
   145                 150                 155                 160
   Ser Thr Gly Ile Asn Ala Asp Asp Lys Thr Val Lys Leu Ile Gly Asp
                   165                 170                 175
   Ile Asn Gln Gln Phe Asp Ile Ser Lys Leu Glu Asp Val Gly Lys Ala
               180                 185                 190
   Val Leu Ser Ile Ala Thr Asn Pro Asn Pro Arg Glu Leu Pro Asp Thr
           195                 200                 205
   Ile Arg Ile Gly Ser Asp Arg Ile Thr Val Lys Asp Val Ile Asp Arg
       210                 215                 220
   Tyr Ser Lys Asp His Asn Val Glu Leu Lys Val Val Ser Glu Gln Ser
   225                 230                 235                 240
   Ala Glu Asp Ala Lys Lys Glu Phe Thr Glu Ser Leu Lys Ala Gly Phe
                   245                 250                 255
   Asp Gly Glu Lys Phe Leu Trp Tyr Leu Gln Val Ile Ala Ala Gln Gly
               260                 265                 270
   Leu Asp Lys Gly Leu Leu Ser Ser Lys Leu Asp Asn Glu Leu Val Asn
           275                 280                 285
   Pro Gly Glu Ser Leu Trp Lys Trp Gly Lys Tyr
       290                 295
<210> 7
   <211> 166
   <212> PRT
   <213> Candida albicans
<400> 7
   Met Ala Ser Ser Val Lys Leu Ala Thr Ala Leu Lys Gln Arg Ala Ile
   1               5                   10                  15
   Leu Thr Lys Glu Leu Ser Glu Leu Asp Asp Lys Ile Gln Ser Ser Leu
               20                  25                  30
   Ile Ser Gln Val Gly Met Lys Lys Ile Asn Asp Pro Asp Lys Leu Tyr
           35                  40                  45
   Leu Asp Tyr Val Ala Lys Ser Gln Glu Leu Ala Lys Leu Val Ser Ser
       50                  55                  60
   Ile Asn Tyr Thr Asn Asn Ile Thr Pro Ile Glu Leu Asp Leu Thr Met
   65                  70                  75                  80
   Gly Lys Tyr Asp Asn Thr Ile Lys Thr Ile Asn Asp Ala Leu Ile Cys
                   85                  90                  95
   Arg Asp Arg Ile Phe Lys Lys Leu Gln Phe Val Lys Lys Ile Ser Thr
               100                 105                 110
   Ala Gly Lys Glu Gln Pro Leu Asp Ser Lys Asp Glu Ile Lys Phe Val
           115                 120                 125
   Ser Phe Ile Asp Val Asp Lys Tyr Asp Thr Leu Ala Gln Glu Leu Asn
       130                 135                 140
   Thr Gln Phe Glu Asn Leu Asn Leu Lys Leu Gln Glu Ile Asn Trp Gln
   145                 150                 155                 160
   Val Asp Leu Val Glu Ile
                   165
<210> 8
   <211> 161
   <212> PRT
   <213> Candida albicans
<400> 8
   Met Lys Leu Ala Glu Ala Leu Asn Leu Lys Lys Asn Leu Glu Arg Asp
   1               5                   10                  15
   Ala Gly Glu Leu Lys Ser Leu Ile Leu Lys Cys Cys Gln Ala Gln Thr
               20                  25                  30
   Gly Glu Asn Pro Pro Phe Asp Pro Asn Glu Leu Phe Glu Gln Tyr Glu
           35                  40                  45
   Glu Ile Asp Lys Leu Ile Thr Asp Ile Thr Ile Lys Ile Gln Arg Thr
       50                  55                  60
   Asn Asn Glu Ile Lys Phe Ala Tyr Asp Asn Asp Asn Lys Ser Asn Glu
   65                  70                  75                  80
   Glu Pro Leu Arg Ser Met Thr Gln Ala Ile Ala Asp Ile Asp Asp Leu
                   85                  90                  95
   Glu Arg Gln Ile Asn Val Thr Asp Asp Ile Ile His Asn Gly Ile Ile
               100                 105                 110
   Thr Lys Ser Tyr Ser Thr Lys Lys Ile Ala Asp Val Ser His Val Asp
           115                 120                 125
   Val Val Ala Tyr Asp Lys Thr Arg Lys Lys Met Asn Glu Arg Leu Asp
       130                 135                 140
   Lys Leu Lys Leu Arg Ile Gln Ser Ala Asn Trp Glu Phe Asp Leu Ile
   145                 150                 155                 160
   Asp
<210> 9
   <211> 6619
   <212> DNA
   <213> Candida albicans
<400> 9
   gttgataaga tctctaatga tgatttgtaa tttgagcgaa tttttatctc ttgttgggtt 60
   tttgtggatg ttgcacataa agctgcaagg acatcaccaa caacaagtag caagtgtggc 120
   tagagttaca aatccgtgta tggtagcaca actgatgaca tttgaataga tgtcatacaa 180
   caaaatatgg aatagttttg gataataaac agcacgtgac tattgttaac cagatggctg 240
   ttgagaagac actaagacag tacaacagat atctacaaac acctataggt aaatgaggac 300
   tgcctatttc cttgaaacca ttttctatta cttatttaca ttagttgtat cttttcatta 360
   attcaatttc attcataaat atcaaatacc tagtatctaa ctacatattg cctactttaa 420
   atgaaaataa accttgggac ataataaatt atatctgata atcttagtac ttgccccatt 480
   tccataaaga ttcacctgga ttaaccaatt cattatccaa tttactggag agtaaacctt 540
   tatctaaacc ttgagcagca ataacttgta aataccataa gaatttatca ccatcaaaac 600
   caactttcaa agattcagta aactctttct tggcgtcttc tgcagattgt tctgaaacaa 660
   ttttcaattc aacattatga tctttagagt atctatcaat tacatcttta acagtaattc 720
   tatcagaacc aattctaatg gtatctggta attctcttgg attaggatta gtagcaatag 780
   aaagtacagc tttaccaaca tcttctaatt tagaaatatc aaattgttga ttaatgtcac 840
   caatgagttt aacagttctg tcttcagcat taataccagt tgaaccaacc cattcataaa 900
   gaaaagctcc tggaacagca aataacgaag ttataatatc aacaacttta actcctgatt 960
   ttctgacatt ttctgaatgt tgagttttaa ttcctaaaaa ccctggagca tattcatcaa 1020
   ctttaggaat atcagtacca aattgtgatg ggataaataa ttttggttta attgcatcaa 1080
   ctaaattttc gatattggcg aaagcctctg gatttggacc aattaattca ataataacat 1140
   cagtaccaga taatttttgg ctcaaagttg atttaattga ttcttcattg atttcagaaa 1200
   caacatattc aattttgtca ttcttagttt ctggttcctt tctggtaatt gccttgattg 1260
   ggaagttgat cttatcatca aaaataccag aattgatagc ttcaagaact ggtttaccta 1320
   agaaaccatt caaaccgatg atagtaattg agactttaga cattgtgata gatagatatt 1380
   atagattaat tattagataa gcttgtgtaa ttgatcaatt gcttgattaa tgagattgga 1440
   aaacaaaaaa ttacaagcca tgttgaatgg aggaaacacg tctatttata atgggtttga 1500
   ttcaatgtga tgcttaatag gggagtgggg ggttatgcaa tgtaaggaga gacgacaaaa 1560
   catacttagc taaaaacaca aacacacatt gttgccatag ttaaatgtgg aattaaatgg 1620
   aacaatcttt tcccgtaaaa tgtaaagaaa ggaggaaaaa catacaccaa gaaattgtgg 1680
   cgtaatctga aattctttgt ttctctcttt ctctgtttaa tttgtaatca aatatttttc 1740
   tcattacata atatgcaagt gatgattaat aatcaatatt tgtttatcag ttatatctat 1800
   ttaatccttg tatttataat ttcataacaa atcaataaca acacccgcta cagccacatc 1860
   acaatcaatt tactggtaac ttatttgtaa tctacatatt acctaagatt gtacagaaat 1920
   tgtttctgct tactaaattg tattggtaat aattctacta tggagtaaat agtgttgcta 1980
   ttataattgt ggctagtgta tatactgata tcaattaact cgtattaata atatattagg 2040
   tgtagcaagc ttgatatctt tgacacagct gttatttgtc tacgccacat tagatttctt 2100
   aaccacaact tgtaaggtag tatagacaac taaaacccta tacagggctc tcttaatcct 2160
   tcctccaatg tctgactact aagtaccatc ataccaccag ggttaggtag atatatagac 2220
   tctcttggat gcccttgtcg tctactccta aagaggttta catactcaac tagaaaatac 2280
   aagctattaa cgtagttaaa gtattacaca actttgtata gctgactttg caaccctcag 2340
   attcgttgaa ttttttttgt aagtaacaat ctgtggtctc tggtaaatag ctcaagcctc 2400
   agttattact aatcttatta cttatactgc caattctaaa accatcagca aattcattat 2460
   taagcattgg ttttagattt catttggtta gtaaagttgc agatacgata tacataacta 2520
   agcatagact ataataatcc gaaactaaaa gttgcaatat tccgatatcc ggtatccttt 2580
   ccgttgaatc ttttgtattc taaatattaa tacaaggtat agcttgatta attgttggtt 2640
   tatgtagatg acagtacttt acttattggt tgtaaataat ctaaagaagt acagaattta 2700
   tttcaaaatt ggtatccaaa ctcaaccaaa ataataaatc aaccacccaa attgatatta 2760
   tctaaaacta caaataaaga tatacaaatg gtttagccta ctgaaagtat acataatagc 2820
   atcagttaac tcctcgtagt tcaaccaact aaaaataacc tttataatgg ttctcatctc 2880
   tagttgtatc attctgttcc tcatcctcgt catcttcatc ttcgtttgaa ttatcttcaa 2940
   cagtatataa cccaattctt cttcgattat tattattgtt atttgatgat agctgtttat 3000
   tctttttaaa tttcttgaaa tttttccttc cagcatatct cgattcagta tcatcatcaa 3060
   cactcttatt tttgttacgt cgatttctag gattaactaa atcaacaatt tccacaattg 3120
   ccaaattttg taaattttca ttaatttcaa attcagattc caaatctaaa tttgatgatt 3180
   tattcaattc ttgaactttt aaaactgcat ctactaatga tacttttggt gtaaattttg 3240
   gaattttaaa tggtttttga tcatcattag tagtttcatc tttattctta cgttttgatc 3300
   cgattgcagg aatttcttcc tcgtcttctt gttcttcttc ttcattttta attgatggaa 3360
   aattggaact agacttttgt gaattggaat ctggtttaga tttccttgct aattgatcag 3420
   ttgatgatga taaattcgaa tcacccaaac tattaagtgc atcttcttgg ttgattttac 3480
   gactttgcgt atgagaattc aacacttttt gattttgctt tatcgttgaa atttttttaa 3540
   tgggatcact taattgtgtt atttcttgag ttattgaatc attatcacca ctggtattaa 3600
   ttttctgact atttaattct ggtgatgtgc ttttattgtc agtgtccaag attgatactt 3660
   taggggatgg atcacgtgta gatggtactg gtgtagttgt agttgttggt gttgtcgtac 3720
   ctaaggagaa gaaatgtaat ctatcaactt tttcatattt ccttctcttt ctccctgaag 3780
   ggtgccgcca cctcaggtgt ttgtgttgta actgatttat catctgatgc taacatagtt 3840
   gttgaggtac gtttcaatta aatcaaataa tttttgggaa gatctagtat tgagccttct 3900
   ttaattgatt gccataattg atcttctgtt agagtttgga aattatgtct attcaaagtt 3960
   gttgccattg tagttggaac aattaaaatg aattgattat cacctatttt cgatttcaat 4020
   acgtcttctt gttgttgtat taacacttta aaatctataa ttattgtttc caaccaattt 4080
   attctctcca atgatactaa tttaacatta tttaatggct ttaatctata acttttcggt 4140
   aaatagtgat catccttttt tggtaagtat ttcttataat ctatatccaa aagccaatcc 4200
   ttaacactat tatacccatc ctcattatca tgacctaata ctttttgtaa ccattgtgaa 4260
   tttaaaattg ggataccctt aataattgcc aatttgaatt catctttttg ttcttcctcc 4320
   ttgatatcgt tattatcact atcataataa taatgagtgg catccattat attatcaacg 4380
   atttgaatat caataccatg atcttttatt attgttttgt aatgattttg atcacatttt 4440
   tcattcgcac taatatataa tttaaaggga atccattcaa tgaacaatgg agtatcttga 4500
   gttattattt gtcctgtttg tttatcaatt tgtggaatag tttcaatttt caatttgaat 4560
   tcatttgtga aatcaatttc ttctggttta ctatcttttg ataataattt atatgttttc 4620
   ccatttataa tcgttttcgc tcgactacat attctaatga ttaataatgt tttatcacta 4680
   cctattgaag aagaagacga tgacgatgat ggtggttggg tattgcctat gttgggtcca 4740
   gttattaatt caatcaattc tcttgatact ttaggtgacc agaattttat atcagtattt 4800
   tcatcacgtc ccacggtata atttgtttct ggtgataaat gtttatattc taatgaattg 4860
   ttagttggcc tggggttaaa taaatatcaa aaatcttcag tttctaacat acctcctctt 4920
   ggatcatctt tatgatatgt tatccacata gttaagtgta catttgcttg tcaatgaagt 4980
   cttcctaagc gaatttgaac tgaaaaaaaa cctatcgcga tccctttaca actttaacaa 5040
   acgtataata aacaggttgc ataaaacctt ctacaaacca ttcagaatct tactttcata 5100
   aatagtgatt tattatgaat cgtttcttta aactagtaat acttgtatca ataatgaatc 5160
   cctaagcttt tatgttgtaa ctggattaat atcagagttg taggcgtggt cacgtgacat 5220
   agatagaata agagtcgaag ggaacaacat taattagact tgatacttat tgtattaaga 5280
   taaatgtgaa tttacaataa caaatggtga atatagttta tcagttcata ctagacgatt 5340
   agttgaaatt tatttatgtt tgtttggata atcgtttgtt ataaaaatag aaatcgtcat 5400
   tttttttttg ctcacttgtc ctgttcattc cattcaaggc tgtaactgta aatgttaaac 5460
   taaatttcat ttcatttcat tcaacaaaaa aatatcatac tctatttaac ttcaacaaac 5520
   taatctcaag aaccagctta cttcctttta taatactaca acaacatata ttatacaact 5580
   aatcaaagat gggtattaag aaaatgtttc aaaagaaaga accaaccgaa caagaaattc 5640
   gtgaagaatt aagtcgagtt ggcatttcta caagatcaaa taatactcga caagagaagt 5700
   ttggtgcatt taaaaattat gctcaagaac gagctaatat gaaaccacaa ttaggaccag 5760
   ttggtggtaa tccttatgcc aatattaatc ctgggaccaa caataataat aataatccat 5820
   atgccaatga taatgggaat aatagtactg gcaaccccaa caacaacaac ggtggtaacc 5880
   cctatggtgg tggtgttact aataataatc cttatggagg ctctggtggt aatggaagag 5940
   gatcatcacc tagtccttat gcaccgacta catcaacaac tactagatca tctaatccat 6000
   atggaaacaa taatggttct agatcaagtc aaaacacttc tagtccttat gccaaatcaa 6060
   ctaacaattc atcatattct aactcaccgt attctggatc aactgtaaat aatggtaatc 6120
   gtggcggcca tagcaacaac agcaatagtt ctgctggtgg taacccttat gctgccggtg 6180
   gtagaagttc acaatctcaa aattcacgag acaatgtata tacagctcct gccactcgta 6240
   catcaactag acaaactcaa ggatatggag gtggtgatac cgattcgact cttgacctta 6300
   atgccattcc atcacatcaa atgtttgata ataagaaacc gatcaaaaga aatcaacaaa 6360
   gttcacaaca acctgccaat gattataatt tagatttaaa tgatgaatat ggcgaagaag 6420
   aagacttgaa tttggatata agtgaagtac ctgaagaaca acaacaaatc aattctgaag 6480
   atgaagaagt agaagccatt aaacaagata ttaaatttgt caaacaagaa tcagttcaaa 6540
   gtaccagaaa tactcttaga atggcacaag aagctgatgc atcgggtact aatactttag 6600
   gaatgttagg atcgcaact                                              6619
<210> 10
   <211> 7965
   <212> DNA
   <213> Candida albicans
<400> 10
   tagctaagta tgctttgtcg tctctcctta cactgcttaa ccctccaccc ctctatttgg 60
   cttcacattg aatcaaacca ttataaatag acgtgtttcc tccattccac atgacttgta 120
   attctttgtt ctccaatctc atcaatcaat tacacaagct tatctaataa ttaatctata 180
   atatctatct atcacaatgt ctaaagtctc aattactatc atcggtttga atggtttctt 240
   aggtaaacca gttcttgaag ctatcaattc tggtattttt gacgataaaa tcaatttccc 300
   aattaaagct attacaagaa aagaaccgga aactaaaaat gacaaaattg aatacgttgt 360
   ttctgaaatc aatgaagaat caattaaatc aaccttgagc caaaaattat ccggtactga 420
   tgttattatt gaattaattg gtccaaatcc agaggctttc gctaatatcg aaaaattaat 480
   tgatgcaatt aaaccaaaat tattcattcc atcacaattt ggtactgata ttcctaaagt 540
   tgatgaatat gctccagggt ttttaggaat caaaactcaa cattcagaaa atgtcagaaa 600
   attaggagtt aaagttgttg atattataac ttcgttattt gctgttccag gagcttttct 660
   ttatgaatgg gttggttcaa ctggtatcaa tgctgatgac aaaactgtta aacttattgg 720
   tgacattaat caacaatttg atatttctaa attagaagat gttggtaaag ctgtactttc 780
   tattgctact aatcctaatc caagagaatt accagatacc attagaattg gttctgatag 840
   aattactgtc aaagatgtca ttgatagata ttctaaagat cataatgttg aattgaaagt 900
   tgtttctgaa caatctgcag aagatgccaa gaaagagttt actgaatctt tgaaagctgg 960
   ttttgatggt gagaaattct tatggtattt acaagttatt gctgctcaag gtttagataa 1020
   aggtttactc tccagtaaat tggacaacga attggtcaac ccaggtgagt ctttatggaa 1080
   atggggcaag tactaagatt atcagatata atttattatg tcccaaggtt tattttcatt 1140
   taaagtaggc aatatgtagt tagatactaa gtatttgtta ttaataaatc agattaaatc 1200
   aatgaaagga ttacaattaa tgtagatgag gattagaaat tggtttctaa tagagaactg 1260
   ttgtactctc ttggtgcctt ctcaacagcc atctggttaa cagtagtcac gtgctgttga 1320
   tcatccaaaa ctattccaca ttttgcttta tgaaaaccta ccctaatatt gccaattgtg 1380
   ctaccataca cggacttgta attccagcca cactttctta cttgttattg ttgatttcct 1440
   tgcagcttta tttgcaacat ccacaaaaac ccaacaagac aaaaataacc tcaaattaca 1500
   aatcatcatt aaagatctta tcaacaacat gtcccctttt tgataattaa caccttaaat 1560
   taacctcttg tggggttgat caaaccttga atttgaaccc taagtgcaag taagaacgaa 1620
   gttaaagcat ccaagtcttg tgaaaaagca acatgtaacg gcattttagt ttcgaaccga 1680
   gttaaaaatt ggaagtaaga ataagagaaa ctgcgtgacg gctgtgcggc atattattta 1740
   aggcaaattg acgatggcaa aaaaaatctt taatttttcc gtcttggtaa ttaaatatcg 1800
   cgaaggcatt cctttaaagt aatgccttta tgaatggtca aaatgcccta ttttaaaatg 1860
   ccttattact ttctattgcg cttatcatga aatttggcta ttactggcac ggcaacaatt 1920
   taatcagagc atctgaaatg tgggtgatta attgcagagg gcggttagat ataattttag 1980
   atggattgat ttgcctaatt aggaatttgt aactttataa acctctttat aatttttcat 2040
   cttttcattt ttttcaaaat aaaatgggtt tttcacagaa gtaaagatat tgttcatgct 2100
   tattgtgcta acctgatgta aatctcttta ttttaatttg acttcttctt caaaccgttg 2160
   aacacggcac caatgaatga taaaattgat gatatttagt taccagcaag caatgatgac 2220
   agtattccca agaagaagag acaatggcta cagcaactgg agtaaaacaa gtagtttagc 2280
   tacagatcta acctttttgt aacaccccaa ccattctctc tttttttgct atattgaaac 2340
   actgattttt cttaaattat ttacccaact cctaatggaa tataacaacg aggtgcttct 2400
   acttctccac atttccaaca ataaaacatg aatcaacatc aatattgttg caattaatca 2460
   aactcttaac atctccacct ccccactaat gatcgatatt atatcaaaac cattggaaat 2520
   ttagtttggg tttcatttga atttcggtca agaaaattaa aagtaaaaaa gaaaaaaaaa 2580
   atttattatt attattcggt tcgatattgc cgcaaaacca aattccatca tcatttcaca 2640
   atataatata aaaagtcttc aatcttacac cttgcaaaaa gtttcaattt ttttttataa 2700
   aatatttatc tatattctaa ttgttacatt tattctttac ttctaatcaa aacaactata 2760
   tatcaatatt atgtttgaag taggtgaaaa atatcctgtt gaaagcagca gtagttcaaa 2820
   tgacatagaa tctcgtggtg ttcaacctat aacatccctc aaagacaata aatcaatagg 2880
   aatgatagag aaagataatg atgatctatc atgtgaacaa tatagtactt gtgatgaagt 2940
   caaaagagat ttaaaagcaa gacatgtttc tatgattgcc attggtggta caataggtac 3000
   agggttattc atatccactg gttctttact tcacaccact ggtccagtaa tgtcattaat 3060
   atcattttta tttgtcacaa ctttagcata ttcagttaca caatcacttg gggaaatgac 3120
   aacatatatc cccgtttctg gatcatttgc ccaatttata actcgttggg tttcaaaaag 3180
   ttgtggtgct gctaatggtt ggttatattg gttttcatgg gccataacat tcgctttaga 3240
   attatcagtt gttggtcaag tcatacaata ttggactgat gctgtaccat tagctggttg 3300
   gatttccatt tttttcgtct tattaactac atttaattta ttcccggtga aatattatgg 3360
   agaggttgaa ttttggattg cttcaactaa agtaattgct attgttgggt ggctcatata 3420
   tgcattttgt atggtttgtg ggctggtaaa aactggacca gttgggttcc gttattggcg 3480
   gaatggatat gcatggggtg atgggatgat agttctgaat aatggtaaat acgccatttc 3540
   tttcattaat ggtcttatca atgctgtttt tactttccaa ggtactgaat tggttgctgt 3600
   tactgcgggt gaagcttctc caagagcaat ccgtagtgca attaaaaaag tcatgttcag 3660
   aattttggta ttctatgtct tgtgtatgct tttcattggt cttttggttc cttacaacga 3720
   tccaaagctt actcaagatg gtggttttac aagaaactct ccattcctta ttgctatgga 3780
   aaattccggt actaaagttt taccacacat tttcaatgca gtgatcgtta caacaattat 3840
   ttcagctggt aattccaatg tttattcggg atcacgtatt ctttacgggt tagcccaagc 3900
   tggtgtagct cctaaatttt tccttaaaac taacaaaggt ggtgttccat attttgctgt 3960
   cttgttcact gcggcatttg gtgcattggg atatttagca tgttcggaag atggtaataa 4020
   agctttcact tggttattga atattattgc cactgctgga ttgatcgctt ggggattcat 4080
   ttctgtgagt catgtcagat tcatgaatgt tcttagaaaa agaggtttaa gtcgagacat 4140
   tttaccttat aaagcttttt tcatgccata tagtgcatat tatgccatta ttattatatt 4200
   cattgttgtg ttgattcaag gtttcacagt gttttgggac ttcaatgcta gtgatttctt 4260
   cactgcctat atatccgtga tattatttgt tgttctttgg attggtttcc actttttctt 4320
   ttacgggttt ggtaaagatt cttttaaatg ggaaaacata ttaatcccat tggatgattg 4380
   tgatattgat tctggtgtta gagatattaa tgatgctgaa tttgatgtac ctgaacctaa 4440
   aaatgtttgg gaaagattct ggttacttat tgcttaatct taatttatat aatttaatac 4500
   ttaatggaca tagagctttt cagcgattat aatagaaact gattatactt atttaattta 4560
   aatctattta caaatttctc tgaagagtgg ggcgccggtt atacaaactc aacaacgtaa 4620
   taattctgtt taccaactct aaccaacaac aatttaccat caatcaaatg attatcaaca 4680
   tcaaataata taacatcatc tggatcctca atttgatttc tgtccaatcc catgtataca 4740
   ccaccagctt taatcaatct tctcatttca ccctttgatt taccaacaat atcagctaat 4800
   aatgcactca atttgatttc ttcatcaggt gaaggcttat ttcttttaaa caaaatacct 4860
   gatcttttaa aattttctat caatttatca gcgcttacat tatcattaaa tggttgatct 4920
   ggagtaggga ataaaaatcc cgtaataaac gccatttcgt caccaacacc aacaccatgg 4980
   atcaaatcaa caacttcacg tgctaaaaca cgttgagcaa tacgtaaacc aggatcactg 5040
   ttatgtttag gtaataattc accttcaatt acattcaagg gcaataatgt gaacactttt 5100
   aataatttgc ccactatatc atctggaaca ttaatgaaat attgatacat ttgataagga 5160
   gtggtcaaac tagaatcaat aaatactgca tttccagcgg atttaccaaa tttctcacca 5220
   ctagaagtag tcaataatgg aacagtaagt ccataagctt catgtttctt gccatgaaat 5280
   ttcttcaaac gtgaaattaa atcaatacca gcagtaatat tcccccattg atcatttcct 5340
   ccaacttgca tattaacatt ttcatccttg tataaatgcc aaaaatcata agcttgtaga 5400
   atctgatagg taaattcatt gaatccaatt ccacccagtt ctaatcttga ttgaatggaa 5460
   tcacgtgcta acatcgaact aactctaata tgtctaccat atgtagccaa aaattccaac 5520
   atcttcacgt tttcccacca tgaagcatta tttaccgatg tagtatcacc gactttttca 5580
   gtcatgggga attgtcttga tttggcatat tctatcccat tactcaaaaa tgtagaaatt 5640
   tgtcgttgga ttttcgtcac attatcttca acttcaactt catcaatctt gttacgctct 5700
   gttttcctcc cacttggatc tccaacaagt ccagtagcac ctccaacaag tccaacaaca 5760
   tcattaccac tcattttgaa atgtaataac accattaatg gtaataaatt acctaaatgt 5820
   agtgatgatg ccgtaggatc agcaccacaa tataatttga atttgtgatt agaaccacgt 5880
   ttagtcaatt tatataaatt atcatcggtt attgattcaa ttaaatgtcg actttgtaaa 5940
   tattctaata atgaattgtc gggattggtt tctggtgtta aatctttggc ttcagtcaat 6000
   tcatagatgg taggaatgat agtgactgga tctcttgcaa ttgttgaatt aaatctagca 6060
   agccttctaa tcaaaggtat gtttctggta tgtgttttca acatattact tgatgtctgg 6120
   ttgaacttct ggttgtcgtt tcttcgattg aattttttct tgtagcttca ttagcgggct 6180
   tatttgctat tcgcggttta atttttaaag aaagccgcaa attcaaatcc aaatccatct 6240
   caagctgaga tttttcttta attttttttt ttttcacttt actgatatca ttctaatcat 6300
   taaacataca aagctcctaa accaatgaca gatcagatta aaggggatga acataaccaa 6360
   gtaaatggta gtaaaaaaag aaagagaaag agaaataaga acaagaaaaa tgacaacaac 6420
   acaccagtag agacttccga accaataccg acacctgttt atgaagatga tatccatcga 6480
   caaaataaaa agttcaaatt caatgaggaa ggggaaatgg agaaacctca agagtcacct 6540
   gaagaggaac aactagaatt agtggcagat caaggggaac cttgcactga agaaccttta 6600
   ccacaacatg aaggtttcga agagattgaa gtaactgacg acatagatga aacagaagaa 6660
   ccagagaatc ttccaacaag gactcaacaa gaaaaacatc aacacggtaa gaataaattt 6720
   aaacaaaagc ttgaattcaa aagaaaaacc gttgtatata aagatcaaga tgatgaagac 6780
   gatgaggaag aaaataatac tttcaatttt tcacaaaatt cgtttcaact tgcagccacc 6840
   gcccaacaat tgttacaaat tagagagaaa ttgcccattt atcatcataa ggataaaatc 6900
   attgaatgca ttaataataa tcaagtcact atcgtcattg gtgaaaccgg ttcaggtaaa 6960
   tcaacacaaa tccctcaatt tttaatgcca gaaaacccaa aaatgattgg cgtgacacaa 7020
   ccaagaagag ttgccgctgc ttctttagca gcaagagtaa gtgaagaata tggatgtaaa 7080
   ttaggtcaag atgttgggta tcaagttaga ttcactaata tgactaacag acaaacaaaa 7140
   ttgaaatatt taactgatgg tatgcttcta cgagaaatca tgcttgatct gaatttgact 7200
   aaatattcaa caattatcct agatgaagcg catgaaagaa ctattttgac tgatttaatc 7260
   atggggtttt tgaaacaaat tattacttct ggtaaaagaa aagatttgaa aatcgtagtt 7320
   atgagtgcta ctttgaatgc cgaattattt agtaatttct ttgataatgc tcctatttta 7380
   tacattgaag gtaaaatgta tccagtttca caattctact tagatgctga atctgaagat 7440
   attgtggata ccatgatcag aagtataatt caaatcaatc ttaatgaacc cgagggggat 7500
   attctttgct tcttacctgg gcaagaggaa attgataatt gtgttaaaag tttagaacaa 7560
   ttagcacctc aactacctag ggaggcacca ttgattgttc ctttaccttt atatgcagct 7620
   ttatcacctg gccaacaatc taaaatattc gaaaaattac ccaagggaag aagaaaagtg 7680
   attttggcga caaatattgc tgaaacatcc attactgttt ctggtgttaa atatgttata 7740
   gattccggat taaggaaaat taaagtttgg aaacataatt taggactttc tacattattg 7800
   actaccccta tttcacaagc ttcagcaaga caaagagccg ggagagcagg tagagaatct 7860
   gaaggtaaag tattcagatt atatcctgaa tctacttata tggcacttcc aaaacaacaa 7920
   gaatctgaaa ttaaaagaaa tgatattatt ttacccagtt ttgac                 7965
<210> 11
   <211> 5158
   <212> DNA
   <213> Candida albicans
<400> 11
   aataattatc attagtcaat tcaacaacta taggagattt agcagcaatc tcttgagagt 60
   tccttcctcg acatctaaca acaacttgga tatttgacat tgatgataat gctgctatga 120
   gtattaattt aactgaaatc acaagatgaa gaatgaaaac aacaacaaca aagagaaaga 180
   gtttggcaac gggagaggaa gagaaagtgt aaacaaaaac aaacaaccat aaaaatttac 240
   accataaaaa aaaattagaa gtcgtgattg aactatatgc aggccactat aagaagatat 300
   taaaactact ctgattgaat gaatgaatga ttatataaat ccctcttttc tctcaactta 360
   tagccttaat caaagaaatc atcgtcatct tcatcatcat cagcagctat atttttccta 420
   ttgaaagtta tttttgtttg ttgcttttgt tgactatctc tacttaatcc atctaatatt 480
   tgcacaatat ctttttcacc aagtttttga tgtatttgac ccattgaata taatttaata 540
   atataatttt ctactgcttg agctctatcg ggtctaacaa tctttacacg acttaatctt 600
   tctctagctt cattagttaa gactcgattt aatatggtta tggtcatatt ctcttgtgcc 660
   agatcttgtg cgccacccga agaagaagaa gatggattgg tactactgcc acctccggca 720
   gcatttcttt gtaattctgc taatcttgct tgtcttatag catttaattc tgcgtcatcc 780
   ataatgtata gttgtgaatg aatggagaag gagtagtatt aaaataatta gtgtggatag 840
   agtagtcagt cagtcagcca agtgaatagg gaagtaagga aaaattttgg tcacatttaa 900
   cacgaacttc ttgatcaaag aagaagaaga agaaattttt tttctgtcat cacgtgcacg 960
   acctttaaat caattgacaa ttcaaaaatt ttgaacaaca acacaacaca actcattctc 1020
   tctttctctt tctctctctc tctctgtgaa aaaaaaaaaa aaagtaaagg acaataaaga 1080
   aatcaaacaa tcaattaaac aaagttaaaa caggaacttt ttctattcaa gttcaaattg 1140
   aaagagaaag agaaatagaa agaaaaaaaa aatttagttc aaattggaat cttgtcttat 1200
   ttagtttcat ttctatatat cttgtcctca tatactatca acatttagat tgatttgaat 1260
   ccagaatcaa caatttcaac aattcttcag attttgatat agtgtattct atttgacata 1320
   ctttactact accaatacag tcacataatt acatatataa atatattaag agtgggtttt 1380
   cggaacattt tcctcctaga tttaatatag aatccctttc ccctaatttt ttttgcatca 1440
   acatttactt aaaaacttca accccaccaa ctcctaaccc taatatttcc cctttctttt 1500
   tttgcatata agactcccac aatgagttca gataaatcaa atttactaaa aaaatacaag 1560
   attgtctttc ttggtgatca aagtgttggt aaaacatcat taatcaccag atttatgtat 1620
   gatacatttg atgaaactta tgctgccacg attggaattg attttttatc gaaaacaatg 1680
   tatttagaag aaggtaaaac cattagatta caattatggg atactgccgg acaagaaaga 1740
   tttcgatcat taataccttc atatattaga gattctcatg ttgcagtaat atgttatgat 1800
   ataaccaata aaaaatcatt tgataatctt gataaatgga ttaaagatgt taaattagaa 1860
   cgaggtgatg atgtaataat agtattagtc ggtaataaac tggatttagc tagtgataaa 1920
   cgacaagtta gtttagatga tgttgaaaat ttacaaatta aaattggtgc taaatttttc 1980
   attgaaactt caactaaagc aaatcataat gttaaattat tatttaaaaa aattgctcaa 2040
   tcattacctg attttaatca agattccaat gataaatcaa atgataataa taataataat 2100
   aataataatc aactggaaac tattgatata actattgata atactgcacc aaatcctcaa 2160
   ggtaccagca catgttgtta gactagaatc ttagtgtaag aactaataaa aaaacagagc 2220
   aatgggtaga taatattcta agtatattaa cagttcatac aacaacaacc cacacacaca 2280
   catatatata ctatatatat atatcactta tttaatcaat tagatcaaat tcccaatttg 2340
   ccgactgtat acgaagtttt aatttatcta atctctcatt catttctttt cttgtcttgt 2400
   catatgcaac cacgtcaaca tgtgacacat cagcaatctt cttggtcgaa tacagttttg 2460
   taataatacc attatgaatt atatcatctg tcacattgat ttgtctttct aaatcatcaa 2520
   tatcagcaat agcttgtgtc attgatcgaa gtggttcttc attagactta ttatcattat 2580
   cataggcaaa ctttatttca ttattggttc gttgtatttt aatagttata tcagtaatta 2640
   atttatcaat ttcttcatat tgttcaaata attcattagg atcaaatgga gggttttcgc 2700
   cagtttgagc ttgacaacat ttaagaatta atgatttaag ttcaccagca tctctttcca 2760
   agttcttttt taaatttaat gcttcagcta atttcatctt ctctcacaaa atataatata 2820
   actcaattat tggttgtaag attatataga ataaagtata tgaaaatgaa aaaaaaatgg 2880
   ggtgagaggt aaatgtatcc gaatttataa ttctgttgat agcggagaaa agtataattt 2940
   tatttttttt ttggtagttc ggttgtagtt cctctttgct ttattcccct atgcaccctg 3000
   gcatacacaa aagtcaattg attgctttct cttgttaagg cttttggggt tgggcttgga 3060
   gtaattgtcg ttgttgttgt tgctgttcct gatacagtgg aatagagata tgactaattg 3120
   gtattggtat gtttatgttt acacaacaca tatgagtcaa cgaaaaatca attggcttga 3180
   tctgacttct cctggaacta aattctataa tttcatcaac aattgtaggc aaatgtagac 3240
   aaatgttgtg gtttcgtcta gctcaatata accatcaagg tttgttaagc ctccttcctt 3300
   tattattttt gcctcttgaa aggcattttt gatgtaacaa agtgattcta caattgttgc 3360
   gagcaaatta ttggcaaaca tcttttgtga aagaatcata accttccatt cgtttgttcg 3420
   ttttgttagc tcattggctg atgggttctt tagttgctat gaatactgct gctctgtttt 3480
   caaaatcctt ttgttgggaa ggttctaccg attgaggttt aacttgtatt atcgtgtaag 3540
   tgtgttcctg actccgaatt tttgtctata aatagaccta gaaaagttca ctttttttca 3600
   aatttttttt tattcccttt ttcttttttc taatcctcat taacaaatca tattcaaaca 3660
   aatcaatcat tttatgcatt gagtcgtatt aattgttgtt tgttggttat agcttgttgg 3720
   ttgattgatt ggttggttgg tagtataaac attttcatta ctctaatggc ctcctcagta 3780
   aagttggcta cggcacttaa acaacgtgct atattgacaa aagaattgtc tgaattagat 3840
   gataaaatac aatcttcatt gattctgcaa gttggtatga aaaaaatcaa tgatccagat 3900
   aaattgtatt tagattatgt tgctaaatct caagaattgg ctaaattggt atcatcaata 3960
   aattatacta ataatataac tccaattgaa cttgatttga caatgggaaa gtatgataat 4020
   actataaaaa caattaatga tgcattaatt tgtcgagacc gaatatttaa aaaattacaa 4080
   tttgtgaaaa aaatatcaac agcaggtaaa gaacaaccat tagattccaa agatgaaatt 4140
   aaatttgtat catttattga tgttgataaa tatgatactt tggcccaaga attaaatact 4200
   caatttgaga atttgaattt gaaattacaa gaaataaatt ggcaagttga tcttgttgag 4260
   atataaaaag gatagtggtg ctggatcgcc attgataata ttctttactt gttactttat 4320
   gtaaaaggat ttaaaaaata ttgttggtac tactcgtttc ctccctccca aatcgaataa 4380
   tagaactata gaaccatatc ccccctataa ttattttatc tgattttatt agttataaag 4440
   tacaaatcta ttatcaattg ttttattatt tagtattttc ctccaaagtt ttgaactttt 4500
   gttttttatg gttctagttc tttattcttg tttttgggga tttagggttg ccgcttgatt 4560
   tgttgaactt taattgatgc tttgtttagg catagtaatc aagaaaagga agataatgaa 4620
   agggtaggga atgagtagga gggcgggttc ggggacaata tacatgtata gttacgtaca 4680
   ttaatgtaaa tatattctta aaattcctag tttgtaaatt aattgatggt gttgttgtct 4740
   ttgtattttt aaagtattca aaaattttga gtcaatttcg ttaccaaatc ttaatgaata 4800
   gtaacacgtc taaccaaatt tcaacaaaaa gtttcatacg accaacaact tatatgcttt 4860
   tcagtatgta tatatcttcc atatttttat ttgtatatga ttgaattgat aattgtaata 4920
   gagttaaaag aatgaagaag aagaagaagt gggtttttgc aaccaacaga acagttaggt 4980
   tattcttgtg tacacgacca gatcaaatat gtatgtgaga gagagacgga aatagaattt 5040
   tctggaaaga aaaaaaaaaa aaaatttcct tcctgttttt ctctcgcccc gtgtgggtgg 5100
   gtctctctca ctgttgtgta attcgtacca acaattccgg agccaaattt ctttcacc   5158
<210> 12
   <211> 968
   <212> DNA
   <213> Candida albicans
<400> 12
   cttctctcac aaaatataat ataactcaat tattggttgt aagattatat agaataaagt 60
   atatgaaaat gaaaaaaaaa tggggtgaga ggtaaatgta tccgaattta taattctgtt 120
   gatagcggag aaaagtataa ttttattttt tttttggtag ttcggttgta gttcctcttt 180
   gctttattcc cctatgcacc ctggcataca caaaagtcaa ttgattgctt tctcttgtta 240
   aggcttttgg ggttggggtt ggagtaattg tcgttgttgt tgttgctgtt cctgatacag 300
   tggaatagag atatgactaa ttggtattgg tatgtttatg tttacacaac acatatgagt 360
   caacgaaaaa tcaattggct tgatctgact tctcctggaa ctaaattcta taatttcatc 420
   aacaattgta ggcaaatgta gacaaatgtt gtggtttcgt ctagctcaat ataaccatca 480
   aggtttgtta agcctccttc ctttattatt tttgcctctt gaaaggcatt tttgatgtaa 540
   caaagtgatt ctacaattgt tgcgagcaaa ttattggcaa acatcttttg tgaaagaatc 600
   ataaccttcc attcgtttgt tcgttttgtt agctcattgg ctgatgggtt ctttagttgc 660
   tatgaatact gctgctctgt tttcaaaatc cttttgttgg gaaggttcta ccgattgagg 720
   tttaacttgt attatcgtgt aagtgtgttc ctgactccga atttttgtct ataaatagac 780
   ctagaaaagt tcactttttt tcaaattttt ttttattccc tttttctttt ttctaatcct 840
   cattaacaaa tcatattcaa acaaatcaat cattttatgc attgagtcgt attaattgtt 900
   gtttgttggt tatagcttgt tggttgattg attggttggt tggtagtata aacattttca 960
   ttactcta                                                          968
<210> 13
   <211> 456
   <212> DNA
   <213> Candida albicans
<400> 13
   atgtccgacg aaagaacttt tattgctatc aaaccagacg gtgttcaaag aggtttaatc 60
   tcatctatct tgggtagatt tgaacaaaga ggtttcaaat tagttggtat taaattggtt 120
   caaccaactg aatctttatt gagaactcat tatgaagatt tacaatctaa accatttttc 180
   ccatctttat tatcttatat gttatccggt ccagtcttag ctactgtttg ggaaggtaaa 240
   gatgttgtta aacaaggtag agccattttg ggtgctacta acccattaca atctgctcca 300
   ggtaccatca gaggtgattt tgccattgat atgggtagaa acgtttgtca tggttctgat 360
   tctgttgaat ctgctaacaa agaaattgac ttgtggttca agaaagaaga attggttgaa 420
   tataaaccag ctttgttcgg ttggatctac gaataa                           456
<210> 14
   <211> 151
   <212> PRT
   <213> Candida albicans
<400> 14
   Met Ser Asp Glu Arg Thr Phe Ile Ala Ile Lys Pro Asp Gly Val Gln
   1               5                   10                  15
   Arg Gly Leu Ile Ser Ser Ile Leu Gly Arg Phe Glu Gln Arg Gly Phe
               20                  25                  30
   Lys Leu Val Gly Ile Lys Leu Val Gln Pro Thr Glu Ser Leu Leu Arg
           35                  40                  45
   Thr His Tyr Glu Asp Leu Gln Ser Lys Pro Phe Phe Pro Ser Leu Leu
       50                  55                  60
   Ser Tyr Met Leu Ser Gly Pro Val Leu Ala Thr Val Trp Glu Gly Lys
   65                  70                  75                  80
   Asp Val Val Lys Gln Gly Arg Ala Ile Leu Gly Ala Thr Asn Pro Leu
                   85                  90                  95
   Gln Ser Ala Pro Gly Thr Ile Arg Gly Asp Phe Ala Ile Asp Met Gly
               100                 105                 110
   Arg Asn Val Cys His Gly Ser Asp Ser Val Glu Ser Ala Asn Lys Glu
           115                 120                 125
   Ile Asp Leu Trp Phe Lys Lys Glu Glu Leu Val Glu Tyr Lys Pro Ala
       130                 135                 140
   Leu Phe Gly Trp Ile Tyr Glu
   145                 150
<210> 15
   <211> 486
   <212> DNA
   <213> Candida albicans
<400> 15
   atgtctcaat tttacgaatt agctccaaaa gacgccaaag gtgaaccata cccatttgaa 60
   caattgaaag ggaaagttgt ccttatcgtc aatgttgctt ccaaatgtgg attcactcct 120
   caatacaagg gtttagaaga attgaataag aaatttgctg atcaaccagt acaaatcttg 180
   ggtttcccat gtaatcaatt tggccaccaa gaaccaggta gtaacgaaga aattggatca 240
   ttctgttcat tgaactacgg tgttacattc ccagtcttgg ataaaattga agtcaatggt 300
   gacaataccg atccagttta taaatatttg aaatcacaaa agagtggtgt tttgggattg 360
   accagaatta aatggaattt tgaaaaattc ttgattgacc aaaatggtaa agttattgaa 420
   agattcagtt cattgactag tccagaaagt atcggtacca agattgaaga attgttgaag 480
   aaataa                                                            486
<210> 16
   <211> 161
   <212> PRT
   <213> Candida albicans
<400> 16
   Met Ser Gln Phe Tyr Glu Leu Ala Pro Lys Asp Ala Lys Gly Glu Pro
   1               5                   10                  15
   Tyr Pro Phe Glu Gln Leu Lys Gly Lys Val Val Leu Ile Val Asn Val
               20                  25                  30
   Ala Ser Lys Cys Gly Phe Thr Pro Gln Tyr Lys Gly Leu Glu Glu Leu
           35                  40                  45
   Asn Lys Lys Phe Ala Asp Gln Pro Val Gln Ile Leu Gly Phe Pro Cys
       50                  55                  60
   Asn Gln Phe Gly His Gln Glu Pro Gly Ser Asn Glu Glu Ile Gly Ser
   65                  70                  75                  80
   Phe Cys Ser Leu Asn Tyr Gly Val Thr Phe Pro Val Leu Asp Lys Ile
                   85                  90                  95
   Glu Val Asn Gly Asp Asn Thr Asp Pro Val Tyr Lys Tyr Leu Lys Ser
               100                 105                 110
   Gln Lys Ser Gly Val Leu Gly Leu Thr Arg Ile Lys Trp Asn Phe Glu
           115                 120                 125
   Lys Phe Leu Ile Asp Gln Asn Gly Lys Val Ile Glu Arg Phe Ser Ser
       130                 135                 140
   Leu Thr Ser Pro Glu Ser Ile Gly Thr Lys Ile Glu Glu Leu Leu Lys
   145                 150                 155                 160
   Lys
<210> 17
   <211> 1080
   <212> DNA
   <213> Candida albicans
<400> 17
   atggctcctc cagcagtttt aagtaaatcc ggtgttatct acggtaaaga cgtcaaagac 60
   ttgtttgact atgctcaaga aaaaggtttt gccattccag ctatcaatgt cacttcatcc 120
   tcaactgttg ttgctgcttt agaagctgcc agagacaaca aggctccaat catcttgcaa 180
   acttctcaag gtggtgctgc ctactttgcc ggtaaaggtg tcgacaacaa agatcaagct 240
   gcttccattg ctggttcaat tgctgccgct cactacatta gagccattgc tccaacttat 300
   ggtatcccag ttgttttaca cactgatcac tgtgccaaaa aattattgcc atggtttgat 360
   ggtatgttga aagccgatga agaattcttt gctaagaccg gtactccatt gttctcatcc 420
   cacatgttgg atttatctga agaaaccgat gacgaaaaca ttgctacttg tgccaaatat 480
   ttcgaaagaa tggctaaaat gggtcaatgg ttagaaatgg aaattggtat cactggtggt 540
   gaagaagatg gtgtcaacaa cgaacacgtt gaaaaagatg ctttatacac ttctccagaa 600
   actgttttcg ctgtctacga atctttacac aagatttctc caaacttttc tattgctgct 660
   gcttttggta acgtccacgg tgtttacaaa ccaggtaatg tgcaattgag accagaaatc 720
   ttgggtgacc accaagttta cgctaagaaa caaattggta ctgatgctaa acacccatta 780
   tacttggttt tccacggtgg ttctggttct actcaagaag aattcaacac tgctatcaag 840
   aatggtgttg tcaaggtcaa cttggacact gattgtcaat acgcttactt gactggtatc 900
   agagattacg tcaccaacaa gattgaatac ttgaaagcac cagttggtaa cccagaaggt 960
   gctgacaaac caaacaagaa atactttgac ccaagagtct gggttagaga aggtgaaaag 1020
   accatgtcca agagaattgc tgaagctttg gatattttcc acaccaaagg acaattgtaa 1080
<210> 18
   <211> 359
   <212> PRT
   <213> Candida albicans
<400> 18
   Met Ala Pro Pro Ala Val Leu Ser Lys Ser Gly Val Ile Tyr Gly Lys
    1               5                  10                  15
   Asp Val Lys Asp Leu Phe Asp Tyr Ala Gln Glu Lys Gly Phe Ala Ile
               20                  25                  30
   Pro Ala Ile Asn Val Thr Ser Ser Ser Thr Val Val Ala Ala Leu Glu
           35                  40                  45
   Ala Ala Arg Asp Asn Lys Ala Pro Ile Ile Leu Gln Thr Ser Gln Gly
       50                  55                  60
   Gly Ala Ala Tyr Phe Ala Gly Lys Gly Val Asp Asn Lys Asp Gln Ala
   65                  70                  75                  80
   Ala Ser Ile Ala Gly Ser Ile Ala Ala Ala His Tyr Ile Arg Ala Ile
                   85                  90                  95
   Ala Pro Thr Tyr Gly Ile Pro Val Val Leu His Thr Asp His Cys Ala
               100                 105                 110
   Lys Lys Leu Leu Pro Trp Phe Asp Gly Met Leu Lys Ala Asp Glu Glu
           115                 120                 125
   Phe Phe Ala Lys Thr Gly Thr Pro Leu Phe Ser Ser His Met Leu Asp
       130                 135                 140
   Leu Ser Glu Glu Thr Asp Asp Glu Asn Ile Ala Thr Cys Ala Lys Tyr
   145                 150                 155                 160
   Phe Glu Arg Met Ala Lys Met Gly Gln Trp Leu Glu Met Glu Ile Gly
                   165                 170                 175
   Ile Thr Gly Gly Glu Glu Asp Gly Val Asn Asn Glu His Val Glu Lys
               180                 185                 190
   Asp Ala Leu Tyr Thr Ser Pro Glu Thr Val Phe Ala Val Tyr Glu Ser
           195                 200                 205
   Leu His Lys Ile Ser Pro Asn Phe Ser Ile Ala Ala Ala Phe Gly Asn
       210                 215                 220
   Val His Gly Val Tyr Lys Pro Gly Asn Val Gln Leu Arg Pro Glu Ile
   225                 230                 235                 240
   Leu Gly Asp His Gln Val Tyr Ala Lys Lys Gln Ile Gly Thr Asp Ala
                   245                 250                 255
   Lys His Pro Leu Tyr Leu Val Phe His Gly Gly Ser Gly Ser Thr Gln
               260                 265                 270
   Glu Glu Phe Asn Thr Ala Ile Lys Asn Gly Val Val Lys Val Asn Leu
           275                 280                 285
   Asp Thr Asp Cys Gln Tyr Ala Tyr Leu Thr Gly Ile Arg Asp Tyr Val
       290                 295                 300
   Thr Asn Lys Ile Glu Tyr Leu Lys Ala Pro Val Gly Asn Pro Glu Gly
   305                 310                 315                 320
   Ala Asp Lys Pro Asn Lys Lys Tyr Phe Asp Pro Arg Val Trp Val Arg
                   325                 330                 335
   Glu Gly Glu Lys Thr Met Ser Lys Arg Ile Ala Glu Ala Leu Asp Ile
               340                 345                 350
   Phe His Thr Lys Gly Gln Leu
           355

## Beschreibung

Die vorliegende Erfindung betrifft Biochips, insbesondere Nucleotid-Chips, die hyphenspezifische Proteine codierende Nucleotidsequenzen enthalten, Protein-Chips, die hyphenspezifische Proteine enthalten, und Antikörper-Chips, die gegen diese hyphenspezifischen Proteine gerichtete Antikörper enthalten, diagnostische Zusammensetzungen, die diese Nucleotid-, Protein- oder Antikörper-Chips enthalten, Verfahren zum Auffinden und Identifizieren von Substanzen, die gegen durch Candida-Arten verursachten Krankheiten therapeutisch wirksam sind, und Verfahren zum Diagnostizieren einer durch Candida verursachten Krankheit.

Zu den Sprosspilzen oder Hefen zählen neben den schon seit langem zum Beispiel in der Lebensmittelindustrie kommerziell genutzten Hefen der Familie Saccharomycetaceae, auch asporogene Hefen wie beispielsweise Hefen der Gattung Candida. Einige Angehörige der Gattung Candida sind in der Lage, Mycelverbände zu bilden, andere vermehren sich lediglich durch Sprossung. Candida albicans ist der am häufigsten isolierte humanpathogene Pilz. Candida albicans verursacht häufig opportunistische Infektionen, also Infektionen durch normalerweise relativ unproblematische Keime bei immunsupprimierten Patienten. Derartige Infektionen nehmen bei diesen Patienten einen schweren Verlauf und verkürzen die Überlebenszeit zum Beispiel HIV-Infizierter oder mittels Chemo- oder Radiotherapie behandelter Krebspatienten entscheidend. Gegenwärtig wird die Behandlung von systemischen Infektionen mit Candida albicans hauptsächlich mittels Azolen oder Polyenen durchgeführt. Die Behandlung mittels dieser beiden Substanzklassen weist jedoch Nachteile auf. Polyene führen zu starken Nebenwirkungen, gegen die Azole entwickeln sich zunehmend Resistenzen (DiDomenico, 1999, Curr Opin Microbiol 2, 509 bis 515, Georgopapadakou, 1998, Curr Opin Microbiol 1,547 bis 557).

Da die klinischen Befunde bei Pilzinfektionen überwiegend uncharakteristisch sind, gestaltet sich die exakte Diagnose pilzlicher Infektionen, insbesondere von Candida-Infektionen, äußerst schwierig. Bei Verdacht auf Candida-Befall der Haut oder der Schleimhäute müssen beispielsweise Oberflächenabstriche abgenommen und mikroskopisch untersucht werden. Bei Befall innerer Organe ist es erforderlich, Organbiopsien histologisch zu untersuchen, um invasives Wachstum nachweisen zu können. Zur Diagnostik einer generalisierten Candida-Infektion ist die Spiegelung des Augenhintergrundes indiziert. Ferner müssen mehrere Blutkulturen untersucht werden, die an aufeinanderfolgenden Tagen venös abzunehmen sind. Bei einer Nierenbeteiligung ist zusätzlich der Urin zu untersuchen. Derartige mikroskopische Nativpräparate erlauben jedoch nur den Nachweis von polymorphen Pilzzellen (Hyphen, Pseudohyphen und Blastosporen) und Sporen, ohne dass jedoch die genaue Spezies ermittelt werden kann und geeignete therapeutische Maßnahmen eingeleitet werden können.

Neben dem mikroskopischen Nachweis ist es daher unerlässlich, Kulturen zur exakten Speziesbestimmung anzulegen. Eine weitere diagnostische Möglichkeit, die jedoch bislang nicht den erhofften Aussagewert hat, ist der Nachweis von Candida-Antigen im Serum des Patienten. Ein hoher Titer spricht zwar für eine systemische Candida-Infektion, ist aber nicht beweisend, während ein negativer Befund eine systemische Infektion nicht ausschließen kann.

Die Entwicklung weiterer verbesserter Diagnostika zur zweifelfreien Zuordnung einer gesundheitlichen Störung zu den durch Vertreter der Familie Candida hervorgerufene Infektionen und von Antimycotica zur Behandlung von durch Vertreter der Familie Candida hervorgerufene Infektionen ist daher dringend erforderlich.

Das der vorliegenden Erfindung zugrundeliegende technische Problem besteht also darin, Mittel und Verfahren zur Diagnose von durch Candida albicans hervorgerufenen Infektionen und zur Entwicklung von Substanzen, die gegen Candida-verursachte Erkrankungen therapeutisch wirksam sind, zur Verfügung zu stellen.

Die Erfindung löst das ihr zugrundeliegende technische Problem durch die Bereitstellung von Biochips, insbesondere eines Nucleotid-Chips, umfassend einen festen Träger und mindestens eine daran fixierte Nucleotidsequenz, welche für die Identifizierung und Transkription eines Gens codierend für ein hyphenspezifisches Protein aus Candida, insbesondere Candida albicans, geeignet ist, wobei diese Nucleotidsequenz ausgewählt ist aus der Gruppe bestehend aus:
(a) einer Nucleotidsequenz, definiert in SEQ ID Nr. 1, 2, 3, 4, 12, 13, 15 oder 17, oder eines komplementären Strangs davon,
(b) einer Nucleotidsequenz, codierend eine Aminosäuresequenz, definiert in SEQ ID Nr. 5, 6, 7, 8, 14, 16 oder 18, oder eines komplementären Strangs davon und
(c) einer Nucleotidsequenz, die mit einer der (a) oder (b) genannten Nucleotidsequenzen hybridisiert.

Im Zusammenhang mit der vorliegenden Erfindung wird unter einem Biochip eine Vorrichtung verstanden, die eine Vielzahl biologischer Substanzen, beispielsweise Nucleotidsequenzen, Proteine oder Antikörper, in immobilisierter oder fixierter Form umfasst und mit deren Hilfe mittels Hybridisierungs- und/oder Bindungsverfahren eine kleine Menge eines Liganden, der unter geeigneten Bedingungen an die biologische Substanz binden kann, in einer kleinen Probe nachgewiesen werden kann. Unter einem Nucleotid-Chip wird eine Vorrichtung verstanden, die eine Vielzahl verschiedener Nucleinsäuren oder Nucleotidsequenzen wie DNA oder RNA in immobilisierter Form enthält und mit deren Hilfe mittels Nucleinsäure-Hybridisierung eine kleine Menge einer komplementären Nucleinsäure in einer kleinen Probenflüssigkeit oder mittels DNA/Protein-Bindungsuntersuchungen eine kleine Menge eines an Nucleinsäuren bindenden Proteins nachgewiesen werden kann.

Die erfindungsgemäßen Nucleotid-Chips enthalten an einem festen Träger fixierte Nucleotidsequenzen, die ausschließlich in der hyphal wachsenden Form von Candida albicans exprimierte Proteine codieren beziehungsweise die ausschließlich die Expression hyphenspezifischer Proteine regulieren. Das heisst, die auf den erfindungsgemäßen Nucleotid-Chips enthaltenen Nucleotidsequenzen werden während des hefeartigen Wachstums von Candida nicht exprimiert. Die erfindungsgemäß beschriebenen Nucleotidsequenzen und die davon codierten Proteine weisen keine signifikanten Homologien, beispielsweise mit Proteinen von Saccharomyces cerevisiae, einem verwandten, nicht-pathogenen, nicht hyphal wachsenden Pilz auf. Es ist bekannt, dass das filamentöse Wachstum, also die Bildung von Hyphen, eine wichtige Voraussetzung für die Ausprägung der Virulenzeigenschaften von Candida ist (Mitchell, 1998, Curr. Opin. Microbiol., 1, 687-692). So sind Candida albicans-Formen, die keine Hyphen ausbilden, im Modellsystem (Mus musculus) avirulent (Lo et al., 1997, Cell 90, 939 bis 949). Im Zusammenhang mit der vorliegenden Erfindung wird daher unter einem hyphenspezifischen Protein ein Protein und/oder Peptid verstanden, das ausschließlich in Arten der Gattung Candida exprimiert wird und vorzugsweise für die Virulenz von Candida, insbesondere Candida albicans Bedeutung hat.

Die erfindungsgemäß verwendeten, spezifisch in der pathogenen Form von Candida albicans vorkommenden Nucleotidsequenzen und die von diesen codierten Proteine stellen daher ausgezeichnete diagnostische Hilfsmittel für das Erkennen lokaler oder systemischer Candidosen dar, insbesondere zum Erkennen lokaler oder systemischer Candida albicans-Infektionen. Darüber hinaus bieten sie die Möglichkeit, im Falle einer Candida-Infektion hyphal wachsende, also virulente Candida albicans-Formen von hefeartig wachsenden, also nicht-virulenten Candida albicans-Formen zu unterscheiden.

Überdies erweisen sich die erfindungsgemäß verwendeten Nucleotidsequenzen und Proteine als besonders wertvoll für die Entwicklung von Medikamenten zur Bekämpfung von Candidosen. Die erfindungsgemäßen Nucleotidsequenzen und Proteine können als Targets für die Identifikation spezifisch auf diese wirkender Substanzen eingesetzt werden. So können etwa Substanzbibliotheken auf die Interaktion der in ihnen vorhandenen Substanzen mit erfindungsgemäßen Proteinen oder erfindungsgemäßen Nucleotidsequenzen hin untersucht werden.

Die Erfindung betrifft daher in bevorzugter Ausführungsform einen vorgenannten Nucleotid-Chip, der eine Nucleotidsequenz umfasst, die eine proteincodierende Nucleotidsequenz ist, ausgewählt aus der Gruppe bestehend aus den Nucleotidsequenzen der SEQ ID Nr. 1, 2, 3, 4, 13, 15 und 17. Diese Nucleotidsequenzen codieren die in SEQ ID Nr. 5, 6, 7, 8, 14, 16 und 18 dargestellten Aminosäuresequenzen. Diese Sequenzen sind besonders hilfreich bei der Diagnose von Erkrankungen, die von Candida-Arten verursacht werden, indem sie den gezielten Nachweis der Gegenwart von Candida in Oberflächenabstrichen oder Organbiopsien ermöglichen. Beispielsweise können die auf einem solchen Nucleotid-Chip enthaltenen erfindungsgemäßen Nucleotidsequenzen mit markierten DNA-Proben, die aus Quellen wie Hautabstrichen, Biosieproben oder eigens angelegten Pilzkulturen isoliert oder mittels PCR-Verfahren amplifiziert wurden, unter stringenten Bedingungen hybridisiert werden. Da die erfindungsgemäßen Nucleotidsequenzen keine signifikanten Homologien mit Nucleotidsequenzen von verwandten Pilzen aufweisen, erlaubt der Nachweis einer Hybridisierung daher den Nachweis von Candida in einer mit Pilzen infizierten Probe. Die erfindungsgemäß verwendeten Nucleotidsequenzen ermöglichen jedoch nicht nur den einfachen Nachweis von Candida, sondern auch den Nachweis, dass Candida in hyphaler Form wächst und dementsprechend virulente Eigenschaften aufweist. Beispielsweise kann aus Hautabstrichen oder Biospieproben gezielt mRNA isoliert und/oder mittels PCR-Verfahren amplifiziert werden. Nach Markierung mit geeigneten Markierungsmitteln wird die so erhaltene mRNA mit dem die erfindungsgemäßen Nucleotidsequenzen enthaltenden Nucleotid-Chip hybridisiert. In W09818927 werden virulenz- und hyphen-assozüerte Proteine von Candida albicans aufgeführt, davon abgeleitet Methoden zur Detektion von Candida albicans, sowie Screening-Methoden zur Detektion von Interaktionen mit bezeichneten Proteinen. Allerdings weist dieses Dokument vom Stand der Technik nicht die in der vorliegenden Anmeldung beschriebenen Nukleotid-, und Aminosäurensequenzen auf, und vermag ferner nicht eine hyphenspezifische Expression nachzuweisen. Da die erfindungsgemäßen Nucleotidsequenzen ausschließlich während des hyphalen Wachstums von Candida transkribiert und exprimiert werden, nicht jedoch während des hefeartigen Wachstums, erlaubt der Nachweis einer Hybridisierung unter Verwendung von isolierter mRNA den Nachweis, dass sich Candida den Übergang in der hyphalen und damit virulenten Wachstumsphase befindet.

Die Erfindung betrifft in einer weiteren bevorzugten Ausführungsform den vorgenannten Nucleotid-Chip, der Nucleotidsequenzen enthält, die regulatorische Elemente von hyphenspezifische Candida-Proteine codierenden Genen darstellen, also Elemente, die insbesondere die Transkription der mit diesen regulatorischen Elementen funktionell verbundenen proteincodierenden Bereiche ermöglichen, beispielsweise Promotoren, Transkriptionsterminationssignale, Silencer, Enhancer usw.. Diese besonders bevorzugten Nucleotidsequenzen können insbesondere Promotoren sein, besonders bevorzugt der in SEQ ID Nr. 12 dargestellte Promotor. Die erfindungsgemäßen regulatorischen Elemente, insbesondere Promotoren, besonders bevorzugt der in SEQ ID Nr. 12 dargestellte Promoter, erweisen sich insofern als besonders vorteilhaft, als sie die für die Induktion von hyphenspezifisch exprimierten Proteinen notwendigen Regulationssequenzen umfassen und dementsprechend verwendet werden können, um weitere spezifische Candida-Proteine zu identifizieren, die in vivo durch Bindung an diese regulatorischen Elemente die Transkription von hyphenspezifischen Proteinen, insbesondere der Proteine mit den in SEQ ID Nr. 5, 6, 7, 8, 14, 16 und 18 dargestellten Aminosäuresequenzen, induzieren oder hemmen können. Nach Identifizierung solcher Proteine können Nucleotid-Chips, die regulatorische Elemente von hyphenspezifisch exprimierten proteincodierenden Nucleotidsequenzen enthalten, verwendet werden, um Substanzen jedweder Art zu identifizieren, die die Wechselwirkung zwischen den regulatorischen Elementen und den daran bindenden Proteinen inhibieren. Unter Verwendung derartiger Nucleotid-Chips ist es also möglich, Substanzen zu identifizieren, die die Expression hyphenspezifischer Proteine hemmen und daher potentiell als spezifisch wirkende Medikamente gegen Candida-Infektionen eingesetzt werden können.

Die Erfindung betrifft in einer weiteren besonders bevorzugten Ausführungsform den vorgenannten Nucleotid-Chip, der als Nucleotidsequenz DNA-, RNA- oder PNA-Sequenzen aufweist. Bei PNA (Peptide Nucleic Acid oder Polyamide Nucleic Acid)-Sequenzen handelt es sich um Moleküle, die nicht negativ geladen sind und in gleicher Weise wie DNA wirken (Nielsen et al., 1991, Science, 254, 1497-1500; Nielsen et al., 1997, Biochemistry, 36, 5072-5077; Weiler et al., 1997, Nuc. Acids Res., 25, 2792-2799). PNA-Sequenzen umfassen ein Polyamid-Grundgerüst aus N-(2-Aminoethyl)-glycin-Einheiten und besitzen keine Glucose-Einheiten und keine Phosphat-Gruppen.

Die erfindungsgemäßen Nucleinsäuremoleküle, die an einem Träger fixiert werden können, können aus natürlichen Quellen, vorzugsweise aus Candida albicans, isoliert werden. Beispielsweise können die Nucleinsäuremoleküle mittels PCR-Verfahren isoliert und amplifiziert werden, wobei doppelsträngige Moleküle erhalten werden. Die erfindungsgemäßen Nucleinsäuremoleküle können aber auch nach bekannten Verfahren in vitro synthetisiert werden, wobei einzelsträngige Oligonucleotide oder Peptid-Oligonucleotide erhalten werden. Durch die Wahl geeigneter Primer können gewünschte Bereiche der erfindungsgemäßen Nucleinsäuren, das heißt sowohl einzelne Bereiche als auch der gesamte Leseraster des Gens, amplifiziert und isoliert werden. Mittels gängiger molekularbiologischer Techniken ist es möglich, verschiedenartige Mutationen in die erfindungsgemäßen Nucleinsäuremoleküle einzufügen. Dadurch können beispielsweise Sequenzvarianten erfasst werden, die in unterschiedlichen klinischen Candida-Isolaten vorkommen. Derartige von der Erfindung erfasste Mutationen können Insertionen, Deletionen, Duplikationen, Inversionen, Additionen, Austausche oder ähnliches sein, auch von ungewöhnlichen Nucleotiden. Auf diese Weise können aber auch modifizierte Oligonucleotide mit funktionellen Gruppen hergestellt werden, die eine kovalente Bindung des Oligonucleotids an das Trägermaterial zur Herstellung des erfindungsgemäßen Nucleotid-Chips ermöglichen. So können beispielsweise Oligonucleotide mit Amino-Modifikationen oder Biotin-Gruppen hergestellt werden, die an auf der Oberfläche des Trägermaterials enthaltenen chemisch reaktiven Gruppen (Epoxide) oder Streptavidin-Gruppen oder Derivate davon kovalent binden können. In einer weiteren bevorzugten Ausführungsform der Erfindung ist vorgesehen, dass Nucleinsäuren mit photolabile Schutzgruppen enthaltenden Nucleosid-Derivaten versehen werden.

Erfindungsgemäß können für den Nucleotid-Chip auch Nucleotidsequenzen verwendet werden, die durch Fusion der erfindungsgemäßen Nucleotidsequenzen mit Genen oder Bestandteilen von Genen aus anderen Quellen erzeugt werden. Erfindungsgemäß können auch verkürzte Nucleotidsequenzen der vorgenannten Art verwendet werden, sofern diese die genannte Hyphenspezifität aufweisen. Erfindungsgemäß ist vorgesehen, dass verkürzte Nucleotidsequenzen eine Länge von mindestens 15 Basenpaaren aufweisen.

In einer weiteren Ausführungsform der Erfindung ist vorgesehen, dass der Nucleotid-Chip auch Nucleotidsequenzen der vorgenannten Art umfasst, die mit den vorgenannten erfindungsgemäßen Nucleotidsequenzen hybridisieren. Hybridisierung bedeutet im Zusammenhang mit diesem Aspekt der Erfindung eine Hybridisierung unter konventionellen Hybridisierungsbedingungen, wie sie in Sambrook et al. (Molecular cloning. A laboratory manual. Cold spring harbor laboratory press, 2. Ausgabe 1989) beschrieben sind, vorzugsweise unter stringenten Bedingungen. Gemäß vorliegender Erfindung spricht man von einer Hybridisierung, wenn nach dem Waschen für eine Stunde mit 1 x SSC und 0,1% SDS bei 55°C, vorzugsweise bei 62°C und besonders bevorzugt bei 68°C insbesondere für eine Stunde 0,2 x SSC und 0,1% SDS bei 55°C, vorzugsweise bei 62°C und besonders bevorzugt bei 68°C noch ein positives Hybridisierungssignal beobachtet wird. Erfindungsgemäß kann eine unter derartigen Waschbedingungen mit einer der in den Sequenzprotokollen angegebenen Nucleotidsequenzen hybridisierende Nucleotidsequenz durch Immobilisierung an den festen Träger für den erfindungsgemäßen Nucleotid-Chip verwendet werden.

Die Identifizierung und Isolierung hybridisierender Nucleotidsequenzen kann beispielsweise unter Verwendung eines erfindungsgemäßen Nucleotid-Chips, der die vorstehend genannten Nucleotidsequenzen oder Teile dieser Moleküle beziehungsweise des komplementären Stranges enthält, erfolgen. Der zur Identifizierung und Isolierung hybridisierender Nucleotidsequenzen eingesetzte Nucleotid-Chip kann beispielsweise Nucleotidsequenzen enthalten, die exakt die oder im wesentlichen die unter SEQ ID Nr. 1 bis 4, 12, 13, 15 oder 17 dargestellte Nucleotidsequenzen oder Teile dieser Sequenzen oder komplementäre Stränge aufweisen. Der Nucleotid-Chip kann aber auch synthetische Fragmente enthalten, die mit Hilfe üblicher Synthesetechniken hergestellt werden und deren Sequenz im wesentlichen mit der einer erfindungsgemäßen Nucleotidsequenz übereinstimmt. Auf diese Weise können Nucleotidsequenzen aus klinischen Candida-Isolaten isoliert und für den erfindungsgemäßen Nucleotid-Chip verfügbar gemacht werden, die gegenüber den in SEQ ID Nr. 1 bis 4, 9 bis 13, 15 oder 17 dargestellten Nucleotidsequenzen Abweichungen oder Mutationen enthalten.

Die mit den erfindungsgemäßen Nucleotidsequenzen hybridisierenden Moleküle umfassen auch Fragmente, Derivate, funktionelle Äquivalente und/oder allelische Varianten der vorstehend beschriebenen Nucleotidsequenzen, die ein erfindungsgemäßes Protein codieren oder dessen hyphenspezifische Expression gewährleisten. Unter "Fragmenten" werden dabei Teile der Nucleotidsequenzen verstanden, die lang genug sind, um das hyphenspezifisch exprimierte Protein zu codieren oder die Hyphenspezifität zu gewährleisten. Der Ausdruck "Derivat", "funktionelles Äquivalent" oder "mutante Abwandlung" bedeutet im Zusammenhang mit der vorliegenden Erfindung, dass sich die Sequenzen dieser Moleküle von den Sequenzen der vorstehend beschriebenen Nucleotidsequenzen an einer oder mehreren Positionen unterscheiden, aber einen hohen Grad an Homologie zu diesen Sequenzen auf der Nucleotidebene aufweisen. Homologie bedeutet dabei eine Sequenzidentität von mindestens 40%, insbesondere eine Identität von mindestens 60%, vorzugsweise über 80%, und besonders bevorzugt, über 90%, 95%, 97% oder 99% auf Nucleinsäureebene.

Die erfindungsgemäßen Nucleotid-Chips umfassen an einem festen Träger fixierte oder immobilisierte Nucleotidsequenzen. Im Zusammenhang mit der vorliegenden Erfindung bedeutet der Begriff "fester Träger" eine unlösliche Matrix. In bevorzugter Ausführungsform besteht der feste Träger aus einem hydrophoben oder schwach hydrophilen Material, wie transparentem Glas, Siliciumdioxid, Metalloxiden, Polymeren und Copolymeren von Dextranen oder Amiden, beispielsweise Acrylamid-Derivaten, Cellulose, Nylon, oder polymeren Materialien, wie Polyethylenterephthalat, Celluloseacetat, Polystyrol oder Polymethylmethacrylat oder einem Polycarbonat von Bisphenol A. Das Trägermaterial wird vorzugsweise vor Fixierung der Nucleotidsequenzen mit einem Oberflächen-aktivierenden Mittel wie Poly-L-Lysin, Polyethylenimin oder Polyalkylamin vorbehandelt, um die Fixierung der Nucleotidsequenzen am Trägermaterial zu verbessern. In einer anderen Ausführungsform wird als Träger verwendetes Glas mit einem Silan-Kupplungsmittel, das eine Amino-Gruppe, eine Aldehyd-Gruppe oder eine Epoxy-Gruppe aufweist, vorbehandelt. Für den erfindungsgemäßen Nucleotid-Chip können jedoch auch die im Handel erhältlichen, bereits beschichteten Trägertypen wie Poly-L-Lysin (Sigma Diagnostics), Super-Aldehyde (Telechem), Super-Amine (Telechem), Silane Prep (Sigma), CMT GAPS (Corning), Type I (Clontech), Type II (Clontech), Arraylink (GeneScan Europe), Type I (Eppendorf), Type II (Eppendorf), Epoxysilan (Quantifoil) und Cast/FastSlides (Schleicher & Schüll) verwendet werden. Weitere geeignete Träger sind solche, die für photolithographisch hergestellte Nucleotid-Chips verwendet werden, beispielsweise die in Lipshutz et al. beschriebenen (Lipshutz, Fodor, Gingeras und Lockhart, 1999, Nat. Genet., 21, 20-24). In besonders bevorzugter Ausführungsform werden für den erfindungsgemäßen Nucleotid-Chip Träger mit Beschichtungen aus Poly-L-Lysin, wie in DeRisi et al. beschrieben (DeRisi, J. L., Iyer, V. R. und Brown, P. O., 1997, Science, 278, 680-686), beispielsweise Poly-Prep Slides (Sigma Diagnostics), oder Aminosilanen, wie Silane-Prep Slides (Sigma), CMT GAPS Slides (Corning) und Super Amine (Telechem), oder Membranen, wie CAST Slides oder FAST Slides (Schleicher & Schüll) verwendet. Zur Immobilisierung amino-modifizierter Oligonucleotide sind epoxy-modifizierte Oberflächen, wie ArrayLink Biochip (GeneScan Europe) oder Epoxysilane Slides (Quantifoil) besonders bevorzugt.

Die Nucleotidsequenzen können über chemische oder photochemische Reaktionen oder durch elektrostatische Wechselwirkungen an das Trägersubstrat gebunden und fixiert werden. In einer bevorzugten Ausführungsform der Erfindung ist vorgesehen, dass die Immobilisierung oder Fixierung der Nucleinsäuren an den verwendeten Trägeroberflächen über eine elektrostatische Bindung oder eine kovalente Bindung erfolgt. Wenn die Nucleinsäuren beispielsweise synthetisch hergestellt wurden und eine funktionelle Gruppe aufweisen, können die Nucleinsäuren an geeignete funktionelle Gruppen auf der Oberfläche des Trägermaterials kovalent gebunden und fixiert werden (Lamture et al., 1994, Nucl. Acids Res., 22, 2121-2125; Guo et al., 1994, Nucl. Acids Res., 22, 5456-5465). Erfindungsgemäß können die Nucleinsäuren auch über Spacer oder ein Vernetzungsmittel, beispielsweise ein bifunktionelles Vernetzungsmittel, auf den Oberflächen-aktivierten Träger kovalent gebunden werden. In einer bevorzugten Ausführungsform der Erfindung ist vorgesehen, dass die Bindung der Nucleotidsequenzen an den Träger im Falle von Polylysin-, Aminosilan- und Membranbeschichteten Nucleotid-Chips mittels UV-Quervernetzung und im Falle von epoxy-modifizierten Chips mittels chemischer Reaktion erfolgt. Die Bindung der Nucleinsäuren an den Träger kann selbstverständlich auch über photochemische Reaktionen erfolgen. Im Falle solcher photolithographisch hergestellten Nucleotid-Chips erfolgt im Anschluss an die Immobilisierung eine gezielte Abspaltung der photolabilen Schutzgruppen mittels Photolyse.

Eine weitere bevorzugte Ausführungsform der vorliegenden Erfindung betrifft daher Verfahren zur Herstellung von Nucleotid-Chips gemäß Anspruch 1, umfassend die Isolierung und/oder Amplifikation von mindestens einer Nucleotidsequenz, die ein hyphenspezifisch exprimiertes Protein von Candida codiert und/oder Regulationselemente dieser Nucleotidsequenz umfasst, oder die chemische Synthese dieser Nucleotidsequenz, die Modifikation der Nucleotidsequenz während oder nach der Synthese oder Amplifikation durch den Einbau funktioneller Gruppen oder Spacer-Einheiten, das Auftragen einer wässrigen Lösung der isolierten oder synthetisierten Nucleotidsequenz auf ein festes Trägermaterial und die Immobilisierung der Nucleotidsequenz an dem Träger mittels chemischer oder photochemischer Reaktion oder elektrostatischer Wechselwirkung.

Eine weitere besonders bevorzugte Ausführungsform der Erfindung betrifft Protein-Chips, umfassend einen festen Träger und mindestens ein daran fixiertes hyphenspezifisches Protein, ausgewählt aus der Gruppe bestehend aus:
(a) einem Protein mit einer Aminosäuresequenz, definiert in SEQ ID Nr. 5, 6, 7, 8, 14, 16 oder 18, oder einem Fragment davon, und
(b) einem Protein mit einer Aminosäuresequenz, die zu einer der in SEQ ID Nr. 5, 6, 7, 8, 14, 16 oder 18 definierten Aminosäuresequenz eine Sequenzidentität von mindestens 80% aufweist, oder einem Fragment davon.

Im Zusammenhang mit der vorliegenden Erfindung wird unter einem Protein-Chip eine Vorrichtung verstanden, die eine Vielzahl verschiedener Proteine oder Peptide in immobilisierter Form enthält und mit deren Hilfe eine kleine Menge eines Liganden, beispielsweise eines Proteins oder eines Antikörpers, das/der an mindestens ein auf dem Träger fixiertes Protein oder Peptid kovalent oder nicht-kovalent binden kann, in einer kleinen Probenflüssigkeit nachgewiesen werden kann.

Die erfindungsgemäßen Protein-Chips enthalten an einem festen Träger fixierte Proteine, die ausschließlich in der hyphal wachsenden Form von Candida albicans exprimiert werden, oder Teile davon. Die erfindungsgemäßen Protein-Chips können daher beispielsweise zum Nachweis von Antikörpern verwendet werden, die im Körper eines Organismus, insbesondere eines Säugers, als Folge einer Immunisierung durch Antigen-Determinanten von hyphal wachsenden Candida-Formen, insbesondere hyphenspezifischen Candida-Proteinen, gebildet wurden. Die Bindung von mindestens einem Antikörper aus Blut, Lymphe, Körpersekreten oder anderen Körperflüssigkeiten eines Organismus an den erfindungsgemäßen Protein-Chip ermöglicht also den Nachweis einer systemischen Candida-Infektion in diesem Organismus, die zur Bildung des gebundenen Antikörpers geführt hat. Darüber hinaus können die erfindungsgemäßen Protein-Chips auch verwendet werden, um beispielsweise solche Proteine aus Candida-infizierten Materialien zu identifizieren und zu isolieren, die in vivo mit den auf dem Protein-Chip enthaltenen Proteinen in Wechselwirkung treten. Nach Identifizierung und Isolierung derartiger interagierender Proteine können die erfindungsgemäßen Protein-Chips auch verwendet werden, um Substanzen jedweder Art zu identifizieren, die die Wechselwirkung zwischen den erfindungsgemäßen Proteinen und damit interagierenden Proteinen hemmen oder fördern können. Unter Verwendung der erfindungsgemäßen Protein-Chips lassen sich also Substanzen detektieren, die potentiell als Medikamente zur Bekämpfung von Candida-Infektionen, insbesondere zur Hemmung des Übergangs vom hefeartigen Wachstum zum hyphalen Wachstum von Candida, geeignet sind.

In einer besonders bevorzugten Ausführungsform der Erfindung ist daher vorgesehen, dass der erfindungsgemäße Protein-Chip neben den hyphenspezifischen Proteinen mit den in SEQ ID Nr. 5, 6, 7, 8, 14, 16 und 18 dargestellten Aminosäuresequenzen auch Derivate, funktionelle Äquivalente oder Varianten dieser Proteine umfassen kann. Im Zusammenhang mit der vorliegenden Erfindung werden unter "Derivaten, funktionellen Äquivalenten und Varianten" insbesondere solche Abkömmlinge der Proteine mit den in SEQ ID Nr. 5 bis 8, 14, 16 und 18 angegebenen Aminosäuresequenzen verstanden, die unter Beibehaltung der Grundstruktur dieser Proteine durch Substitution von Atomen oder Molekülgruppen erhalten werden und deren Aminosäuresequenzen sich von den in SEQ ID Nr. 5 bis 8, 14, 16 und 18 angegebenen Aminosäuresequenzen an mindestens einer Position unterscheiden und die im wesentlichen einen hohen Grad an Homologie auf Aminosäureebene aufweisen. Der dem Fachmann bekannte Begriff "Homologie" bezeichnet den Grad der Verwandtschaft zwischen zwei Polypeptiden, der durch das Ausmaß der Übereinstimmung zwischen diesen Polypeptiden bestimmt wird. Dabei kann eine Übereinstimmung sowohl eine identische Übereinstimmung, also Sequenzidentität, als auch einen konservativen Aminosäureaustausch bedeuten. Vorzugsweise besitzen erfindungsgemäß verwendete Derivate, Varianten oder funktionelle Äquivalente eine Sequenzidentität zu jeweils einer in SEQ ID Nr. 5 bis 8, 14, 16 und 18 angegebenen Aminosäuresequenzen von mindestens 80%, vorzugsweise 85% und besonders bevorzugt von über 90%, 95%, 97% und 99% auf Aminosäureebene. Die Abweichungen zu den in SEQ ID Nr. 5, 6, 7, 8, 14, 16 oder 18 dargestellten Aminosäuresequenzen können beispielsweise durch mit technischen Mitteln erzeugte Deletionen, Substitutionen, Insertionen, Additionen, Austausche oder Rekombinationen der die Aminosäuresequenzen codierenden Nucleotidsequenzen entstanden sein. Es kann sich dabei aber auch um natürlicherweise auftretende Variationen handeln, beispielsweise um auf natürliche Weise entstandende Aminosäuresequenz-Änderungen. Derivate oder Varianten der erfindungsgemäßen hyphenspezifischen Proteine können beispielsweise aus klinischen Isolaten von Candida stammen .

Solche Derivate, funktionelle Äquivalente oder Varianten können sich von den Proteinen mit den in SEQ ID Nr. 5, 6, 7, 8, 14, 16 und 18 dargestellten Aminosäuresequenzen beispielsweise durch eine veränderte Stabilität, Spezifität, ein modifiziertes Temperatur-, pH-Wert- und/oder Konzentrationsprofil, eine veränderte Aktivität und/oder ein verändertes Effektorenmuster unterscheiden. Derivate, funktionelle Äquivalente oder Varianten können auch in anderen Konformationen vorkommen oder andere Untereinheiten beziehungsweise prä- und/oder posttranslationelle Modifikationen aufweisen. Trotz der möglicherweise vorhandenen Unterschiede besitzen die hyphenspezifischen Proteine mit den in SEQ ID Nr. 5, 6, 7, 8, 14, 16 und 18 dargestellten Aminosäuresequenzen und Derivate, Varianten oder funktionellen Äquivalente davon jedoch bestimmte gemeinsame Charakteristika, wie Aktivität, Molekulargewicht, immunologische Reaktivität, Konformation und/oder physikalische Eigenschaften, wie das Laufverhalten in Gelelektrophorese und deren Löslichkeit und andere.

Umfasst ein Protein-Chip Fragmente der Proteine mit den in SEQ ID Nr. 5 bis 8, 14, 16 und 18 angegebenen Aminosäuresequenzen oder der Proteine, deren Aminosäuresequenz eine Sequenzidentität von mindestens 80% zu einer der in SEQ ID Nr. 5, 6, 7, 8, 14, 16 oder 18 definierten Aminosäuresequenz aufweist, werden unter "Fragmenten" insbesondere diejenigen isolierten Bereiche eines Proteins verstanden, die weniger Aminosäuren als das native Protein aufweisen, deren Länge jedoch ausreicht, dass das isolierte Fragment zumindest eine der für das native Protein charakteristischen Funktionen, wie Bindungsvermögen an ein zweites Protein, eine spezifische katalytische Aktivität etc. ausüben kann. Umfasst das Fragment eines Proteins einen Protein-Bereich, der eine Antigen-Determinante oder ein Epitop darstellt, ist es in besonderem Maße zur Bindung eines Antikörpers geeignet.

Die erfindungsgemäßen hyphenspezifischen Proteine, insbesondere die Proteine mit den in SEQ ID Nr. 5, 6, 7, 8, 14, 16 und 18 dargestellten Aminosäuresequenzen, die auf dem erfindungsgemäßen Protein-Chip immobilisiert sind, können aus natürlichen Quellen, beispielsweise aus Candida-infizierten Geweben oder eigens angelegten Candida-Kulturen unter Verwendung üblicher, auf dem Fachgebiet bekannter Verfahren isoliert und aufgereinigt worden sein. Die verwendeten Proteine oder Fragmente können auch synthetischen Ursprungs sein. Beispielsweise lassen sich mit Hilfe des Verfahrens von Merrifield (1985, Angew. Chem., 97, 801) Peptide, also Fragmente der erfindungsgemäßen Proteine synthetisch herstellen. In besonders bevorzugter Ausführungsform der Erfindung können die hyphenspezifischen Proteine oder Peptide mittels üblicher DNA-Rekombinationstechniken hergestellt werden. Beispielsweise können die erfindungsgemäßen proteincodierenden Nucleotidsequenzen, wie die in SEQ ID Nr. 1, 2, 3, 4, 13, 15 und 17 dargestellten Sequenzen oder damit hybridisierende Sequenzen, unter Verwendung üblicher molekularbiologischer und gentechnischer Verfahren in geeigneten Vektoren insertiert und cloniert werden. Vorzugsweise erfolgt die Insertion der erfindungsgemäßen proteincodierenden Nucleotidsequenzen so, dass sie unter der Kontrolle regulatorischer Elemente stehen, das heißt mit diesen operativ verbunden sind. Diese regulatorischen Elemente gewährleisten die Transkription und Synthese translatierbarer Nucleinsäuremoleküle in pro- und/oder eukaryotischen Zellen. Bei regulatorischen Elementen kann es sich um Promotoren, Enhancer, Operatoren, Silencer und/oder Transkriptionsterminationssignale usw. handeln. Nach Transformation in geeignete Wirtszellen, beispielsweise prokaryotische oder eukaryotische Zellen, wie Bakterien-, Hefe-, Pflanzen-, Insekten- oder Säugerzellen, können diese Wirtszellen in einem geeigneten Kulturmedium unter solchen Bedingungen kultiviert werden, die die Bildung des von der proteincodierenden Nucleotidsequenz codierten hyphenspezifischen Proteins oder eines Fragments davon erlauben. Anschließend kann das Protein oder Fragment davon unter Verwendung geeigneter Verfahren aus der Wirtszelle oder dem Medium, in dem die Wirtszelle kultiviert wurde, isoliert und aufgereinigt werden. Zur Herstellung der hyphenspezifischen Proteine oder Fragmente können aber auch geeignete in vitro-Transkriptions-/Translationssysteme eingesetzt werden. In einer besonders bevorzugten Ausführungsform der Erfindung erfolgt die Herstellung der hyphenspezifischen Proteine oder Fragmente davon in bakteriellen Expressionssystemen, wobei die Proteine vorzugsweise als GST-Fusionsproteine, HIS-Tag-Fusionsproteine, pMAL-Fusionsproteine usw. erhalten werden.

Als fester Träger für die erfindungsgemäßen Protein-Chips können die gleichen Materialien verwendet werden, wie vorstehend für die erfindungsgemäßen Nucleotid-Chips beschrieben, beispielsweise Glas, Siliciumdioxid, andere Silica-Materialien, polymere Materialien, wie Fluorpolymere, oder Metalloxide. Vorzugsweise werden diese Trägermaterialien vor der Immobilisierung der Proteine vorbehandelt, beispielsweise mit Silan-Kupplungsmitteln. In einer bevorzugten Ausführungsform der Erfindung werden die von Joos et al. beschriebenen Epoxy-modifizierte Träger oder Membranen (Joos et al., 2000, Electrophoresis, 21, 2641-2650) verwendet.

Erfindungsgemäß ist vorgesehen, dass die Bindung und Immobilisierung der hyphenspezifischen Proteine oder Fragmente am Trägermaterial mittels chemischer oder photochemischer Reaktion oder elektrostatischer Wechselwirkung erfolgt. Die erfindungsgemäßen hyphenspezifischen Proteine und Fragmente davon können beispielsweise durch eine Vielzahl üblicherweise verwendeter funktioneller Gruppen und/oder Spacer oder chemischer Vernetzungsmittel, wie bifunktioneller Vernetzungsmittel, am Trägermaterial gebunden und immobilisiert werden. Eine Übersicht über geeignete funktionelle Gruppen, die eine Bindung von Proteinen an silanisierte Oberflächen ermöglichen, findet sich beispielsweise in Weetall, 1996, Advances in Molecular and Cell Biology, Bd. 15A, 161-192, JAI Press Inc. Falls das zu immobilisierende Protein als GST-Fusionsprotein vorliegt, kann die Bindung des Proteins an den Träger über auf der Trägeroberfläche vorhandene GSH-Einheiten erfolgen. Die Immobilisierung eines pMAL-Fusionsproteins kann über MBP-Einheiten auf der Oberfläche des Trägermaterials erfolgen. Liegt das zu immobilisierende Protein als HIS-Tag-Fusionsprotein, kann die Immobilisierung über Ni²⁺⁻Nitrilotriacetic Acid-Oberflächen (Ni-NTA) erfolgen (Adachi et al., Proc. Nat. Acad. Sci. USA, 97, 7243-7247).

Eine weitere bevorzugte Ausführungsform der vorliegenden Erfindung betrifft daher Verfahren zur Herstellung von Protein-Chips, umfassend die Isolierung von mindestens einem hyphenspezifisch exprimierten Candida-Protein aus einer geeigneten Quelle oder die chemische Synthese oder rekombinante Herstellung dieses Proteins oder eines Fragments davon, die Modifikation des Proteins oder Fragments während oder nach der Isolierung, Synthese oder Herstellung durch den Einbau funktioneller Gruppen oder Spacer-Einheiten, das Auftragen einer wässrigen Lösung des isolierten oder synthetisierten Proteins auf ein festes Trägermaterial und die Immobilisierung des Proteins an dem Träger mittels chemischer oder photochemischer Reaktion oder elektrostatischer Wechselwirkung.

Eine besonders bevorzugte Ausführungsform der vorliegenden Erfindung betrifft einen Antikörper-Chip, umfassend einen festen Träger und mindestens einen daran fixierten Antikörper, der spezifisch gegen ein Protein mit der in SEQ ID Nr. 5, 6, 7, 8, 14, 16 oder 18 dargestellten Aminosäuresequenz oder ein Fragment davon oder ein Derivat davon gerichtet ist.

Da die auf dem erfindungsgemäßen Antikörper-Chip fixierten Antikörper spezifisch gegen hyphenspezifische Candida-Proteine gerichtet sind, kann unter Verwendung eines derartigen Chips die Gegenwart von hyphal wachsenden Candida-Zellen in pilzinfizierten Haut- oder Schleimhautabstrichen, Organbiopsien oder Körperflüssigkeiten nachgewiesen werden. Beispielsweise können aus den vorstehend genannten Proben Proteine extrahiert und nach Markierung mit dem erfindungsgemäßen Antikörper-Chip inkubiert werden. Die Bindung mindestens eines markierten Proteins an den erfindungsgemäßen Antikörper-Chip zeigt daher an, dass in der untersuchten Probe hyphal wachsende Candida-Formen vorliegen.

Im Zusammenhang mit der vorliegenden Erfindung wird unter einem "Antikörper" ein Polypeptid verstanden, das durch ein oder mehrere Immunglobulin-Gene codiert wird und spezifische Strukturen auf einem Antigen, insbesondere eine Antigen-Determinante oder ein Epitop, erkennt und spezifisch daran binden kann. Der Begriff "Antikörper" umfasst nicht nur ein vollständiges Immunglobulin, sondern auch eine Reihe von Fragmenten, die mittels Spaltung mit verschiedenen Peptidasen erhältlich sind. Der Begriff "Antikörper" umfasst auch modifizierte Antikörper, wie oligomere, reduzierte, oxidierte und markierte Antikörper. "Antikörper" umfasst auch Antikörper-Fragmente, die sowohl durch Modifikation intakter Antikörper als auch unter Verwendung von DNA-Rekombinationstechniken erzeugt wurden. Im Zusammenhang mit der vorliegenden Erfindung umfasst "Antikörper" insbesondere auch solche Fragmente wie Fab, F(ab')₂ oder Fvm, die an eine Antigen-Determinante binden können. Das Fab-Fragment kann durch Spaltung des intakten Antikörpers mit dem Enzym Papain erzeugt werden, wobei eine intakte leichte Kette mit einem Teil einer schweren Kette erhalten wird. F(ab')₂ kann durch Behandlung des intakten Antikörpers mit Pepsin ohne anschließende Reduktion erzeugt werden. Bei F(ab')₂ handelt es sich um ein aus zwei Fab'-Fragmenten bestehendes Dimer. Bei Fv handelt es sich um ein gentechnisch hergestelltes Antikörper-Fragment, das den variablen Bereich der leichten Kette und den variablen Bereich der schweren Kette umfasst. Verfahren zur Herstellung solcher Fragmente sind beispielsweise von Harlow und Lane in "Antibodies: A Laboratory Manual", 1988, Cold Spring Harbor Laboratory, New York, beschrieben.

Der Ausdruck "Antikörper, der spezifisch gegen ein Protein gerichtet ist" oder "Antikörper, der spezifisch an ein Protein bindet" bedeutet, dass ein Antikörper unter definierten Immuntest-Bedingungen eine Antigen-Determinante oder ein Epitop eines Proteins erkennen und mittels seines Paratops daran binden kann. Antigen-Determinanten bestehen meist aus chemisch aktiven Molekül-Gruppen, wie Aminosäuren oder Zuckerseitenketten, auf der Oberfläche eines Antigens, beispielsweise eines Proteins, und besitzen eine charakteristische dreidimensionale Struktur. Unter definierten Bedingungen bindet ein Antikörper daher vorzugsweise nur an ein bestimmtes Protein, während an andere Proteine in der gleichen Probe keine nennenswerte Bindung erfolgt.

Ein erfindungsgemäß auf einem Antikörper-Chip immobilisierter Antikörper kann daher an ein Protein, Peptid, Kohlenhydrat, Proteoglycan und/oder einen Lipidkomplex binden, das/der mit den erfindungsgemäß verwendeten hyphenspezifischen Protein in spezifischer Beziehung steht. Ein erfindungsgemäß verwendeter Antikörper kann auch gegen Strukturen gerichtet sein, die als posttranslationelle Modifikation der hyphenspezifischen Proteine anzusehen sind.

Erfindungsgemäß ist vorgesehen, dass der Antikörper-Chip sowohl monoclonale als auch polyclonale Antikörper enthält, die in der Lage sind, spezifisch eine Struktur eines erfindungsgemäßen hyphenspezifischen Proteins zu identifizieren und gegebenenfalls zu binden.

Die auf dem erfindungsgemäßen Antikörper-Chip enthaltenen monoclonalen und polyclonalen Antikörper können unter Verwendung von auf dem Fachgebiet wohlbekannten Verfahren hergestellt und isoliert werden. Die Verfahren zur Herstellung monoclonaler Antikörper unter Verwendung der Hybridom-Technologie sind beispielsweise in Schreyer et al., "Hybridoma Techniques" (1980) oder in den US-Patenten Nr. 4,341,761, Nr. 4,399,121, Nr. 4,472,500 beschrieben.

Als Trägermaterial zur Immobilisierung der Antikörper können die vorstehend für Protein-Chips genannten Materialien, wie Glas, Siliciumdioxid, Nylon, Acrylamid-Derivate, Silica-Materialien, polymere Materialien, wie Fluorpolymere, Metalloxide, usw. verwendet werden. Diese Trägermaterialien werden vorzugsweise vor Immobilisierung der Antikörper vorbehandelt, beispielsweise mit Silan-Kupplungsmitteln. In einer bevorzugten Ausführungsform der Erfindung werden für die Antikörper-Chips die von Joos et al. beschriebenen Epoxy-modifizierte Träger oder Membranen (Joos et al., 2000, Electrophoresis, 21, 2641-2650) verwendet.

Erfindungsgemäß ist vorgesehen, dass die Bindung der gegen hyphenspezifische Proteine gerichteten Antikörper an den Träger über eine chemische oder photochemische Reaktion oder über elektrostatische Wechselwirkungen erfolgt. Wie vorstehend für die Protein-Chips beschrieben, können die erfindungsgemäß verwendeten Antikörper beispielsweise mit Hilfe einer Vielzahl üblicherweise verwendeter funktioneller Gruppen und/oder Spacer oder chemischer Vernetzungsmittel, wie bifunktioneller Vernetzungsmittel, am Trägermaterial gebunden und immobilisiert werden.

Eine weitere bevorzugte Ausführungsform der vorliegenden Erfindung betrifft daher Verfahren zur Herstellung von Antikörper-Chips, umfassend die gentechnische Herstellung, Isolierung oder Synthese mindestens eines gegen ein hyphenspezifisches Candida-Protein gerichteten Antikörpers oder Fragments davon, die Modifikation des Antikörpers oder Fragments während oder nach der Isolierung, Synthese oder Herstellung durch den Einbau funktioneller Gruppen oder Spacer-Einheiten, das Auftragen einer wässrigen Lösung des isolierten oder synthetisierten Antikörpers auf ein festes Trägermaterial und die Immobilisierung des Antikörpers an dem Träger mittels chemischer oder photochemischer Reaktion oder elektrostatischer Wechselwirkung.

Eine weitere besonders bevorzugte Ausführungsform der Erfindung betrifft einen Antikörper-Chip, umfassend einen festen Träger und mindestens einen daran fixierten Antikörper, der spezifisch gegen einen Antikörper gegen ein Protein mit der in SEQ ID Nr. 5, 6, 7, 8, 14, 16 oder 18 dargestellten Aminosäuresequenz gerichtet ist. Ein derartiger Antikörper-Chip umfasst also Antikörper, die gegen die vorgenannten Antikörper gerichtet sind und diese spezifisch erkennen und binden können. Die Verwendung eines derartigen Antikörpers ermöglicht also den Nachweis von Antikörpern gegen hyphenspezifische Proteine von Candida im Blut, der Lymphe, in Körpersekreten oder anderen Körperflüssigkeiten eines Organismus und somit den Nachweis einer systemischen Candida-Infektion in diesem Organismus, die zur Bildung der in den Proben enthaltenen Antikörper geführt hat.

Die vorliegende Erfindung betrifft auch eine diagnostische Zusammensetzung, umfassend mindestens einen erfindungsgemäßen Nucleotid-Chip, einen erfindungsgemäßen Protein-Chip und/oder einen erfindungsgemäßen Antikörper-Chip. Die Erfindung umfasst daher auch Diagnostikkits, die die erfindungsgemäßen Biochips, das heißt Nucleotid-Chips, Protein-Chips und Antikörper-Chips, geeignete Puffersysteme und geeignete Markierungssysteme enthalten.

Die Erfindung betrifft in einer besonders bevorzugten Ausführungsform Verfahren zur Diagnose von durch Candida-Arten verursachten Krankheiten, insbesondere durch Candida albicans verursachte Krankheiten, wobei eine zu testende Probe in einem geeigneten Medium mit einem erfindungsgemäßen Nucleotid-Chip, einem erfindungsgemäßen Protein-Chip und/oder einem erfindungsgemäßen Antikörper-Chip in Kontakt gebracht wird und eine Interaktion zwischen der zu testenden Probe und mindestens einem der genannten Bio-Chips nachgewiesen wird. Die erfindungsgemäßen Verfahren zur Diagnose von Candida-Erkrankungen basieren auf dem Nachweis der Anwesenheit von Nucleotidsequenzen, Proteinen, Antikörpern oder Fragmenten davon, die im Zusammenhang mit den erfindungsgemäßen hyphenspezifischen Proteinen stehen, in Proben, wie Haut- oder Schleimhautabstrichen, Organbiopsien, Körpersekreten, Blut, Lymphe oder anderen Körperflüssigkeiten etc., die pilzlichen Befall zeigen oder im Verdacht stehen, infiziert zu sein.

Im Zusammenhang mit der vorliegenden Erfindung werden unter "durch Candida-Arten verursachten Krankheiten, Krankheitszuständen oder Infektionen" solche Erkrankungen verstanden, die ausschließlich von Candida-Arten, insbesondere jedoch von Candida albicans verursacht werden. Der Begriff umfasst daher auch alle Erkrankungen, die von Candida-Arten wie C. tropicalis, C. krusei, C. parapsilosis und C. guilliermondii, oder Torulopsis (Candida) glabrata verursacht werden. Erfindungsgemäß werden unter diesem Begriff auch Krankheiten oder Krankheitszustände verstanden, die primär andere Ursachen haben und bei denen die Candida-Arten am Gesamtkrankheitsbild nur beteiligt sind beziehungsweise zusätzliche Symptome hinzufügen, zum Beispiel opportunistische Infektionen. Der Begriff "durch Candida-Arten verursachten Krankheiten, Krankheitszuständen oder Infektionen" umfasst insbesondere solche Erkrankungen wie Candida-Mykosen oder Candidosen, die sich im wesentlichen in drei Hauptformen unterteilen lassen. Die erste Hauptform von Candidosen ist durch eine saprophytäre Besiedlung der Haut und Schleimhäute, insbesondere in den äußeren Genitalien, im Mund, Nase-Rachenraum und im Verdauungstrakt gekennzeichnet. Die zweite Candidosen-Hauptform umfasst Infektionen von Haut und Schleimhäuten und wird in erheblichem Maße durch Faktoren wie Schwangerschaft, Diabetes mellitus, schwere Erkrankungen oder Traumen, Cytostatika- und Antibiotikatherapie sowie Alkoholkrankheit begünstigt. Die dritte Hauptform umfasst tiefe Organmykosen bei immunsupprimierten Patienten mit zellulärer Immunschwäche, insbesondere im Bereich der Atemwege, seltener als Candida-Endocarditis, Candida-Meningitis, Candida-Nephritis und Candida-Endophthalmitis.

Aufgrund der Hyphenspezifität und der damit verbundenen Korrelation zu einem Candida-verursachten Krankheitsbild weist die Anwesenheit von Nucleotidsequenzen, Proteinen, Antikörpern oder Fragmenten davon, die im Zusammenhang mit den erfindungsgemäßen hyphenspezifischen Proteinen stehen, auf eine Candida-verursachte Krankheit hin. Der Nachweis der vorgenannten Substanzen erfolgt durch Bindung und/oder Hybridisierung an mindestens einen der erfindungsgemäßen Biochips, die die nachzuweisenden Substanzen spezifisch erkennen.

Sollen im Zusammenhang mit den hyphenspezifischen Proteinen stehende Nucleotidsequenzen in einer Probe nachgewiesen werden, erfolgt deren Nachweis durch Hybridisierung mit dem erfindungsgemäßen Nucleotid-Chip. Dazu werden Nucleinsäuren, beispielsweise DNA oder mRNA, aus einer Probe oder aus einer eigens angelegten Kultur isoliert und/oder amplifiziert, beispielsweise mittels PCR-Verfahren. Die extrahierten Nucleinsäuren werden anschließend markiert, zum Beispiel mit Fluoreszenzfarbstoffen, Enzymen oder radioaktiven Gruppen. Hybridisiert die extrahierte und markierte DNA mit dem Nucleotid-Chip, so zeigt dies die Anwesenheit von Candida in der untersuchten Probe. Hybridisiert die extrahierte mRNA mit dem erfindungsgemäßen Nucleotid-Chip, so weist dies darauf hin, dass die Probe hyphal wachsende Candida-Formen enthält.

Sollen hyphenspezifische Candida-Proteine in einer Untersuchungsprobe nachgewiesen werden, werden Proteine aus der Probe extrahiert und markiert und anschließend mit dem erfindungsgemäßen Antikörper-Chip, der Antikörper gegen hyphenspezifische Proteine enthält, inkubiert. Die Bindung mindestens eines markierten Proteins am erfindungsgemäßen Antikörper-Chip weist auf die Anwesenheit hyphal wachsender Candida-Zellen hin. Der Nachweis von Antikörpern gegen Candida-Proteine in Körperflüssigkeiten kann sowohl unter Verwendung des erfindungsgemäßen Protein-Chips als auch unter Verwendung des erfindungsgemäßen Antikörper-Chips, der Antikörper enthält, die gegen die Antikörper gegen hyphenspezifische Proteine gerichtet sind, erfolgen.

Die vorliegende Erfindung betrifft auch Verfahren zum Auffinden und identifizieren von Substanzen, die gegen Candida-verursachte Krankheiten therapeutisch wirksam sind, wobei eine zu testende Substanz in einem geeigneten Medium mit einem erfindungsgemäßen Nucleotid-Chip, einem erfindungsgemäßen Protein-Chip oder einem erfindungsgemäßen Antikörper-Chip in Kontakt gebracht und eine Wechselwirkung zwischen der zu testenden Substanz und mindestens einem der genannten Chips nachgewiesen wird. So können die erfindungsgemäßen Nucleotid- und Protein-Chips, wie vorstehend beschrieben, verwendet werden, um Substanzen, beispielsweise Proteine, zu identifizieren, die in vivo an Nucleotidsequenzen, die hyphenspezifisch exprimierte Proteine codieren beziehungsweise die Expression dieser Proteine regulieren, oder an die hyphenspezifischen Proteine selbst binden. Solche bindenden Substanzen, insbesondere Proteine, können potentiell als Medikamente gegen Candida-verursachte Krankheiten geeignet sein, wenn sie beispielsweise in der Lage sind, durch Bindung an regulatorische Nucleotidsequenzen die Transkription hyphenspezifischer Proteine zu hemmen oder zu unterbinden oder wenn sie durch Bindung an die hyphenspezifischen Proteine deren Aktivität hemmen oder unterbinden können. Wenn solche Substanzen, die an erfindungsgemäße Nucleotid- oder Proteinchips binden, die Transkription der hyphenspezifischen Proteine induzieren oder fördern oder die Aktivität hyphenspezifischer Proteine derartiger Proteine begünstigen, können die erfindungsgemäßen Biochips verwendet werden, um weitere Substanzen zu identifizieren, die die Interaktion zwischen einem hyphenspezifischen Protein beziehungsweise der dieses codierenden Nucleotidsequenz und der daran bindenden Substanz, insbesondere eines bindenden Proteins, beeinflussen oder hemmen können. Auch solche Substanzen sind potentiell als Medikamente zur Behandlung Candida-verursachter Krankheiten geeignet.

Die vorliegende Erfindung wird durch das folgende Sequenzprotokoll und die folgenden Figuren und Beispiele erläutert.

Das Sequenzprotokoll ist Teil dieser Beschreibung und enthält die Seauenzprotokolle SEQ ID Nr. 1 bis 18. Jede der nachstehend aufgeführten Aminosäuresequenzen wurde aus der entsprechenden DNA-Sequenz abgeleitet und dann teilweise durch Sequenzierung der isolierten Proteine verifiziert.

SEQ ID Nr. 1 stellt die codierende Cap33a DNA-Sequenz aus Contig4-2149 dar.

SEQ ID Nr. 2 stellt die codierende Cap33b DNA-Sequenz aus Contig4-2501 dar.

SEQ ID Nr. 3 stellt die codierende Cap18p DNA-Sequenz aus Contig4-2069 dar.

SEQ ID Nr. 4 stellt die codierende Cap19p DNA-Sequenz aus Contig4-2069 dar.

SEQ ID Nr. 5 stellt die Aminosäuresequenz von Cap33a dar.

SEQ ID Nr. 6 stellt die Aminosäuresequenz von Cap33b dar.

SEQ ID Nr. 7 stellt die Aminosäuresequenz von Cap18p dar.

SEQ ID Nr. 8 stellt die Aminosäuresequenz von Cap19p dar.

SEQ ID Nr. 9 stellt die gesamte DNA-Sequenz von Contig4-2149 dar.

SEQ ID Nr. 10 stellt die gesamte DNA-Sequenz von Contig4-2501 dar.

SEQ ID Nr. 11 stellt die gesamte DNA-Sequenz von Contig4-2069 dar.

SEQ ID Nr. 12 stellt den Promoter-Bereich von Cap18p und Cap19p dar.

SEQ ID Nr. 13 stellt die Cap15p codierende DNA-Sequenz aus Contig5-3226 dar.

SEQ ID Nr. 14 stellt die Aminosäuresequenz von Cap15p dar. Bei Cap15p handelt es sich wahrscheinlich um eine Nucleosid-Diphosphat-Kinase.

SEQ ID Nr. 15 stellt die Cap20p codierende DNA-Sequenz aus Contig4-2178 dar.

SEQ ID Nr. 16 stellt die Aminosäuresequenz von Cap20p dar. Bei Cap20p handelt es sich wahrscheinlich um eine Glutathionperoxidase.

SEQ ID Nr. 17 stellt die Cap40p codierende DNA-Sequenz aus Contig5-2806 dar.

SEQ ID Nr. 18 stellt die Aminosäuresequenz von Cap40p dar. Bei Cap40p handelt es sich wahrscheinlich um eine Fructose-Biphosphat-Aldolase.

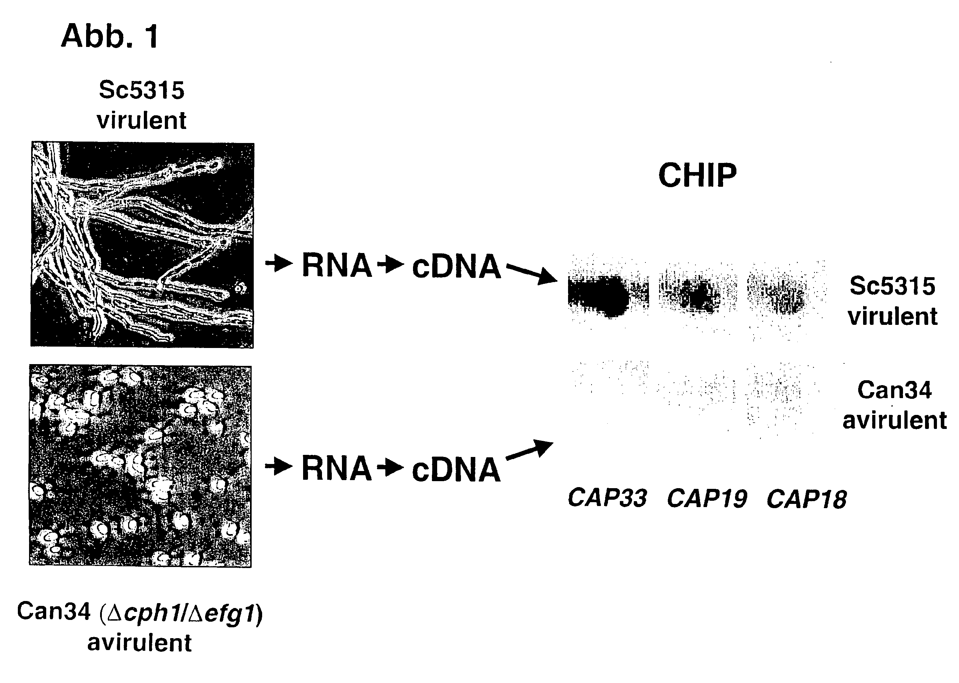

Figur 1 zeigt im linken Teil mikroskopische Aufnahmen des virulenten, hyphal wachsenden Candida albicans-Stammes Sc5315 und des avirulenten, hefeartig wachsenden Candida-Stammes Can34 (Δcph1Δefg1). Aus beiden Stämmen wurde RNA isoliert und in cDNA umgeschrieben. Die markierte cDNA wurde mit einem erfindungsgemäßen Nucleotid-Chip, auf dem CAP33, CAP19 und CAP18 codierende Nucleotidsequenzen fixiert waren, hybridisiert. Die Ergebnisse dieser Hybridisierung sind im rechten Teil von Figur 1 zu sehen. Während die cDNA aus dem avirulenten Stamm mit keiner der auf dem Nucleotid-Chip enthaltenen Nucleotidsequenzen hybridisierte, zeigte die cDNA des virulenten Stammes mit allen drei immobilisierten Nucleotidsequenzen starke Hybridisierungssignale. Wird der C. albicans-Stamm Sc5315 jedoch unter Bedingungen kultiviert, unter denen keine Hyphen entstehen, wird dasselbe Ergebnis wie für den avirulenten Stamm Can34 (Δcph1Δefg1) erhalten.

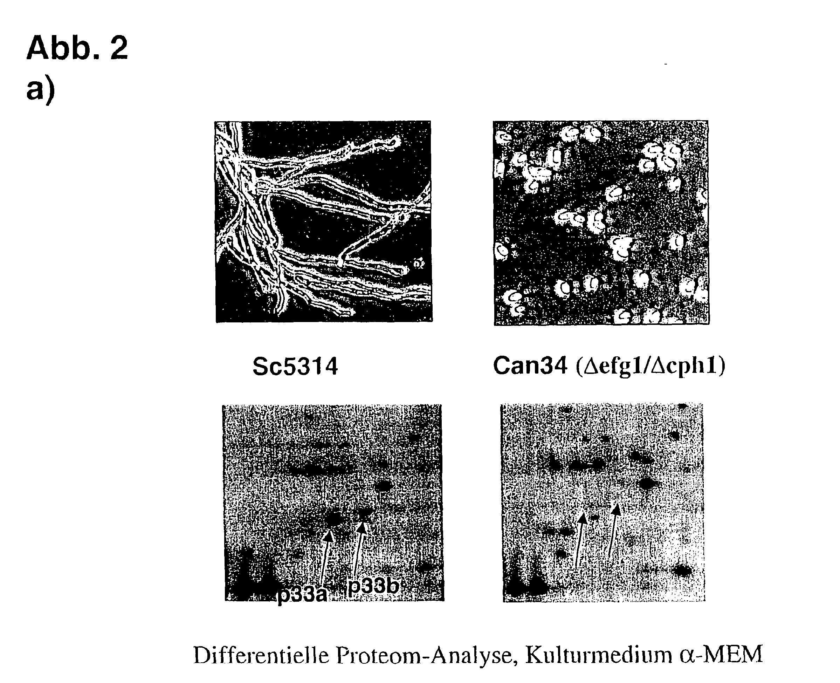

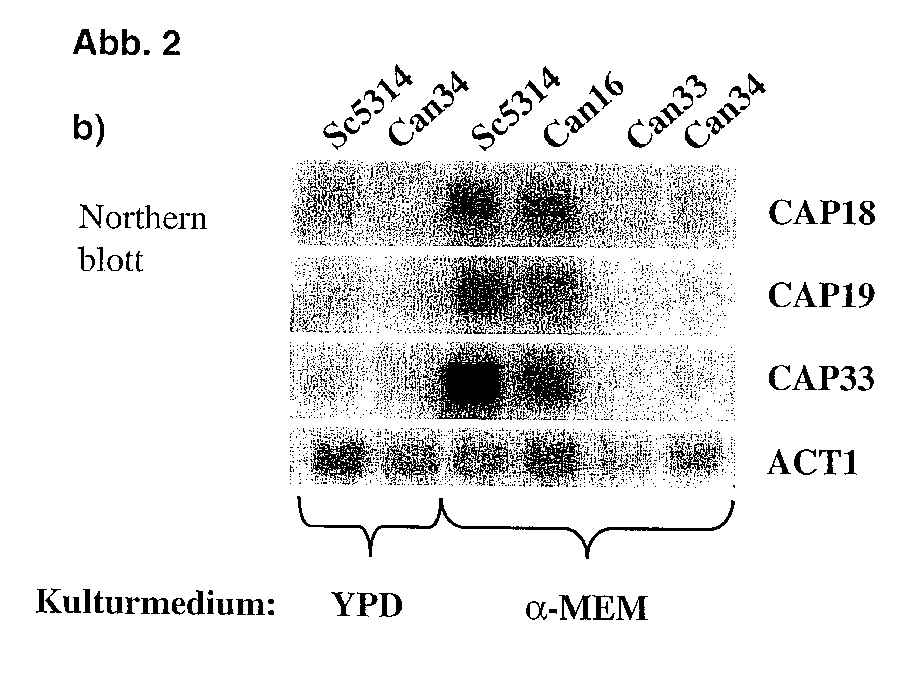

Figur 2a zeigt mikroskopische Aufnahmen des virulenten, hyphal wachsenden Candida albicans-Stammes Sc5315 und des avirulenten, hefeartig wachsenden Candida-Stammes Can34 (Δcph1Δefg1). Darunter sind Abbildungen differentieller Proteom-Analysen der beiden Stämme nach Kultivierung in α-MEM-Medium gezeigt. Aus diesen Abbildungen geht hervor, dass Sc5314 bei Kultivierung in α-MEM-Medium die Proteine p33a und p33b exprimiert, Can34 (Δcph1Δefg1) jedoch nicht.

Figur 2b zeigt die Ergebnisse einer Northern-Blot-Analyse unter Verwendung von RNAs aus diesen beiden Stämmen, die zuvor entweder in YPD-Medium oder in α-MEM-Medium kultiviert worden waren. Ebenfalls einbezogen wurden RNAs aus dem virulenten Stamm Can16 (Δcph1) und dem Stamm Can33 (Δefg1), der keine Hyphenbildung zeigt und eine stark verminderte Virulenz besitzt, wobei beide Stämme vor RNA-Extraktion in α-MEM-Medium kultiviert worden waren. Bei Hybridisierung mit cap18-, cap-19 und cap33-spezifischen Sonden wurden Hybridisierungssignale mit den aus in α-MEM-Medium kultivierten Stämmen Sc5314 und Can16 isolierten RNAs gefunden. Die aus dem in YPD-Medium kultivierten Stamm Sc5314 isolierte RNA zeigte hingegen keine Hybridisierungssignale. Die RNA aus dem avirulenten Stamm Can34 (kultiviert entweder in YPD- oder in α-MEM-Medium) zeigte ebenfalls keine Hybridisierungssignale. Das gleiche Ergebnis wurde auch bei der RNA erhalten, die aus dem in α-MEM-Medium kultivierten Stamm Can33 isoliert worden war. Als Kontrolle wurde eine Aktin-spezifische Sonde (ACT1) eingesetzt.

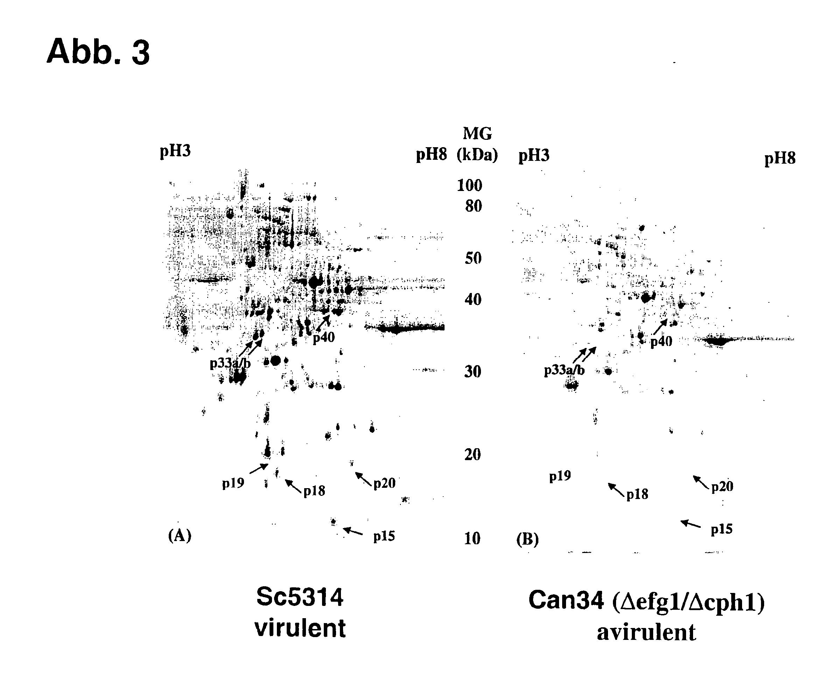

Figur 3 zeigt die Ergebnisse von 2D-Gelelektrophoresen von Proteinextrakten aus den in α-MEM-Medium kultivierten Stämmen Sc5315 und Can34 (Δcph1Δefg1). Während Sc5315 die hyphenspezifischen Proteine p33a, p33b, p40, p15, p18, p19 und p20 exprimiert, werden diese Proteine in Can34 (Δcph1Δefg1) nicht exprimiert.

Weitere vorteilhafte Ausgestaltungen der Erfindung sind in den Unteransprüchen beschrieben.

Die Erfindung wird anhand des folgenden Beispiels näher beschrieben.

### Beispiel 1:

### Die Isolierung der Proteine

Die Proteine wurden aus dem klinischen Isolat Sc5314 durch differenzielle 2D-Gelelektrophorese wie folgt isoliert:

Zur Isolierung der Proteine wurden der virulente Candida albicans-Stamm Sc5314 und der avirulente Candida albicans Stamm Can34 (HLC69) (Lo et al., 1997, Cell, 90, 939-949) gleichzeitig in Vollmedium (YPD: 20g/l Bacto-Pepton; 10g/l Hefe-Extrakt; 0,15g/l L-Tryptophan) über Nacht angezogen, in α-MEM Medium (#22571 Life Technologies/Gibco) mit 2 % Glucose inocculiert (1:100) und für 24 h bei 37°C auf einem Rundschüttler inkubiert. Die so gewonnenen Zellen wurden pelletiert und in einem nicht detergenzhaltigen salinen Puffer (PGSK-Puffer: 0,52g/l NaH₂PO₄•H₂O; 8,8g/l Na₂HPO₄•2H₂O; 2,8g/l NaCl; 0,372g/l KCl; 11g/l Glucose) mit Glasperlen aufgeschlossen. Der daraus isolierte Proteinextrakt wurde mittels isoelektischer Fokusierung und danach mittels SDS-PAGE aufgetrennt. Die Gele wurden mit Silber (vgl. Figur 2a) oder Coomassie (vgl. Figur 2b) angefärbt. Die Proteinspots, die nur in einem der beiden Gele sichtbar waren, wurden aus den coomassiegefärbten Gelen ausgestochen und deren Sequenz bestimmt. Es konnte gezeigt werden, dass die identifizierten Proteine ausschließlich in Sc5314 in α-MEM gebildet werden (Figuren 2a und 3).

Aufgrund der Aminosäure-Sequenz, die durch Edmann-Abbau von tryptischen Fragmenten des Proteins eindeutig zu bestimmen war, konnte die zugehörige DNA-Sequenz über Datenbankvergleiche identifiziert werden. Die DNA-Sequenzen sowie die flankierenden Bereiche wurden durch PCR aus genomischer DNA von Sc5314 amplifiziert und cloniert. Weiterhin wurde die entsprechende DNA-Sequenz aus genomischen Bibliotheken (Liu et al., 1995, Science, 266, 1723-1726) mittels Hybridisierung der durch PCR gewonnenen radioaktiv markierter Fragmente isoliert.

Die codierenden Sequenzen zu jedem der sieben identifizierten Proteine wurden aus den clonierten PCR-Fragmenten -mittels PCR- entfernt und durch Selektionsmarker (URA3) ersetzt (Fonzi and Irwin, 1993, Genetics, 134, 717-728). Diese Konstrukte werden zur Deletion der codierenden Sequenz in C. albicans verwendet. Weiterhin wurden die offenen Leserahmen zu allen sieben Proteinen sowie die Terminationssequenzen mittels PCR isoliert und in Vektoren mit regulierbaren PCK1- und MET3-Promotoren (Leuker et al., Gene, 1997, 19, 192(2), 235-40; Care et al., Mol Microbiol., 1999, 34(4), 792-8.) zur Expression in C. albicans beziehungsweise mit regulierbaren GAL1-10- und MET25-Promotoren (Mumberg et al., Nucleic Acids Res. 1994, 25, 22 (25), 5767-8.) zur Expression in S. cerevisiae und in geeignete Vektoren (pMAL, PGEx etc.) zur Expression der Proteine in Bakterien cloniert.

### Beispiel 2:

### Nachweis der hyphenspezifischen Expression der erfindungsgemäßen Proteine

Die Regulation der hyphenspezifisch exprimierten Proteine findet auf Transkriptionsebene statt, da die mRNA zu allen sieben Proteinen ausschließlich in α-MEM gewachsenen Sc5314-Kulturen nachweisbar ist, nicht jedoch in Sc5314, der in Vollmedium kultiviert wurde, oder im avirulenten Stamm Can34 (Δcph1Δefg1), der in Vollmedium oder α-MEM-Medium kultiviert wurde.

Figur 2b zeigt als Beispiel eine Northern-Analyse von RNA aus den in Vollmedium (YPD oder α-MEM) kultivierten Stämmen Sc5314 und Can34 (Δcph1Δefg1). Bei dieser Northern-Analyse wurde zudem RNA der in α-MEM-Medium kultivierten Stämme Can16 (Δcph1) und Can33 (Δefg1) (Lo et al., 1997, Cell, 90, 939-949) aufgetragen. Can16 (Δcph1) wurde als Stamm beschrieben, der eine mit Sc5314 vergleichbare Virulenz aufweist und Hyphenbildung zeigt. Can33 (Δefg1) zeigt hingegen keine Hyphenbildung und seine Virulenz ist stark reduziert (Lo et al., 1997, Cell, 90, 939-949).

Im virulenten Stamm Sc5314, der in α-MEM-Medium kultiviert wurde, können mit Cap33-, Cap18- und Cap19-spezifischen Sonden die entsprechenden mRNAs nachgewiesen werden. Hingegen lassen sich in Sc5314, der in YPD-Medium kultiviert wurde, mit den vorstehend genannten Sonden keine entsprechenden mRNAs nachweisen. Im virulenten Stamm Can16, kultiviert in α-MEM-Medium, können unter Verwendung der Sonden die entsprechenden mRNAs ebenfalls nachgewiesen werden. Der avirulente Stamm Can34 (Δcph1Δefg1), kultiviert entweder in YPD-Medium oder in α-MEM-Medium, enthält keine entsprechende mRNAs. Auch der in α-MEM-Medium kultivierte Stamm Can33 enthält keine entsprechenden mRNAs.

Die Ergebnisse dieser Northern-Blot-Analyse zeigen einerseits die hyphenspezifische Expression der Proteine mit SEQ ID Nr. 5, 6, 7, 8, 14, 16 und 18 in den virulenten Stämmen Sc5314 und Can16. Andererseits zeigen diese Ergebnisse, dass die hyphenspezifische Expression dieser Proteine hauptsächlich auf der Transkriptionsebene reguliert wird. Dies ist für die Verwendung als diagnostisches Mittel bedeutsam, da die Anwesenheit von mRNA, die im Zusammenhang mit einem der vorstehend genannten hyphenspezifischen Proteinen steht, in einer zu testenden Probe ein Hinweis auf das Vorkommen hyphal wachsender virulenter Candida-Formen in dieser Probe ist.

Proteinextrakte aus den Stämmen Sc5314 und Can34 (Δcph1Δefg1), die in α-MEM-Medium kultiviert worden waren, wurden entsprechend üblichen Verfahren einer 2D-Gelektrophorese unterworfen. Wie in Figur 3 zu sehen, sind die hyphenspezifisch exprimierten Proteine p15, p18, p19, p20, p33a, p33b und p40 ausschließlich im hyphal wachsenden Stamm Sc5314 nachweisbar, nicht jedoch im avirulenten Stamm Can34 (Δcph1Δefg1). In Figur 2a ist darüber hinaus dargestellt, dass der in α-MEM-Medium kultivierte Stamm Sc5314 die Proteine Cap33a und Cap33b produziert, der Stamm Can34 jedoch nicht.

### Beispiel 3:

### Nachweis von hyphenspezifische Proteine codierenden Nucleotidsequenzen mittels eines Nucleotid-Chips

Auf einer Poly-L-Lysin-beschichteten Trägeroberfläche wurden Nucleotidsequenzen, die die hyphenspezifischen Proteine CAP33, CAP19 und CAP18 codieren, fixiert. Der so erhaltene Nucleotid-Chip wurde unter üblichen Hybridisierungsbedingungen mit cDNAs hybridisiert, die aus mRNA des hyphal wachsenden Candida albicans-Stammes Sc5315 und des avirulenten, hefeartig wachsenden Candida-Stammes Can34 (Δcph1Δefg1) hergestellt worden waren. Die Ergebnisse dieser Hybridisierung sind in Figur 1 dargestellt. Während die cDNA aus dem avirulenten Stamm mit keiner der auf dem Nucleotid-Chip enthaltenen Nucleotidsequenzen hybridisierte, zeigte die cDNA des virulenten Stammes mit allen drei immobilisierten Nucleotidsequenzen starke Hybridisierungssignale. Dieser Versuch zeigt, dass unter Verwendung eines erfindungsgemäßen Nucleotid-Chips spezifische Nucleinsäuren nachgewiesen werden können und dass damit hyphal wachsende virulente C. albicans-Stämme von nicht-hyphal wachsenden, avirulenten C. albicans-Stämmen unterschieden werden können.

## Patentansprüche

1. Nucleotid-Chip, umfassend einen festen Träger und mindestens eine daran fixierte Nucleotidsequenz, welche für die Identifizierung und Transkription eines ein hyphenspezifisches Protein codierenden Gens geeignet ist, ausgewählt aus der Gruppe bestehend aus:
(a) einer Nucleotidsequenz, definiert in SEQ ID Nr. 1, 2, 3, 4, 12, 13, 15 oder 17, oder eines komplementären Strangs davon,
(b) einer Nucleotidsequenz, codierend die Aminosäuresequenz, definiert in SEQ ID Nr. 5, 6, 7, 8, 14, 16 oder 18, oder eines komplementären Strangs davon und
(c) einer Nucleotidsequenz, die mit einer der (a) oder (b) genannten Nucleotidsequenzen hybridisiert.

2. Nucleotid-Chip nach Anspruch 1, wobei die Nucleotidsequenz eine DNA-, RNA- oder PNA-Sequenz ist.

3. Protein-Chip, umfassend einen festen Träger und mindestens ein daran fixiertes hyphenspezifisches Protein, ausgewählt aus der Gruppe bestehend aus:
(a) einem Protein mit einer Aminosäuresequenz, definiert in SEQ ID Nr. 5, 6, 7, 8, 14, 16 oder 18, und
(b) einem Protein mit einer Aminosäuresequenz, die zu einer der in SEQ ID Nr. 5, 6, 7, 8, 14, 16 oder 18 definierten Aminosäuresequenz eine Sequenzidentität von mindestens 80% aufweist.

4. Antikörper-Chip, umfassend einen festen Träger und mindestens einen daran fixierten Antikörper, der spezifisch gegen ein Protein mit der SEQ ID Nr. 5, 6, 7, 8, 14, 16 oder 18 gerichtet ist.

5. Antikörper-Chip nach Anspruch 4, wobei der Antikörper ein monoclonaler, polyclonaler und/oder modifizierter Antikörper ist.

6. Antikörper-Chip, umfassend einen festen Träger und mindestens einen daran fixierten Antikörper, der spezifisch gegen einen Antikörper gegen ein Protein mit der SEQ ID Nr. 5, 6, 7, 8, 14, 16 oder 18 gerichtet ist.

7. Diagnostische Zusammensetzung, enthaltend einen Nucleotid-Chip nach Anspruch 1 oder 2, einen Protein-Chip nach Anspruch 3 oder einen Antikörper-Chip nach einem der Ansprüche 4 bis 6.

8. Verfahren zum Diagnostizieren einer durch Arten der Gattung Candida verursachten Krankheit, wobei eine zu testende Probe in einem geeigneten Medium mit einem Nucleotid-Chip nach Anspruch 1 oder 2, einen Protein-Chip nach Anspruch 3 oder einen Antikörper-Chip nach einem der Ansprüche 4 bis 6 in Kontakt gebracht wird und eine Interaktion zwischen der zu testenden Probe und mindestens einem der genannten Chips nachgewiesen wird.

9. Verfahren zum Auffinden und Identifizieren therapeutisch gegen durch Arten der Gattung Candida verursachten Krankheiten wirksamer Substanzen, wobei eine zu testende Substanz in einem geeigneten Medium mit einem Nucleotid-Chip nach Anspruch 1 oder 2, einen Protein-Chip nach Anspruch 3 oder einen Antikörper-Chip nach einem der Ansprüche 4 bis 6 in Kontakt gebracht wird und eine Interaktion zwischen der zu testenden Substanz und mindestens einem der genannten Chips nachgewiesen wird.

## Claims

1. Nucleotide chip comprising a solid support and at least one nucleotide sequence fixed thereto, which is suitable for the identification and transcription of a gene encoding a hypha-specific protein, selected from the group comprising:
(a) a nucleotide sequence defined in SEQ ID No. 1, 2, 3, 4, 12, 13, 15 or 17, or a complementary strand thereof,
(b) a nucleotide sequence encoding the amino acid sequence defined in SEQ ID No. 5, 6, 7, 8, 14, 16 or 18, or a complementary strand thereof, and
(c) a nucleotide sequence, which hybridises with one of the nucleotide sequences specified in (a) or (b).

2. Nucleotide chip according to Claim 1, wherein the nucleotide sequence is a DNA, RNA or PNA sequence.

3. Protein chip comprising a solid support and at least one hypha-specific protein fixed thereto, selected from the group comprising:
(a) a protein with an amino acid sequence defined in SEQ ID No. 5, 6, 7, 8, 14, 16 or 18, and
(b) a protein with an amino acid sequence, which has a sequence identity of at least 80% to an amino acid sequence defined in SEQ ID No. 5, 6, 7, 8, 14, 16 or 18.

4. Antibody chip comprising a solid support and at least one antibody fixed thereto, which is specifically directed to a protein with the SEQ ID No. 5, 6, 7, 8, 14, 16 or 18.

5. Antibody chip according to Claim 4, wherein the antibody is a monoclonal, polyclonal and/or modified antibody.

6. Antibody chip comprising a solid support and at least one antibody fixed thereto, which is specifically directed to an antibody against a protein with the SEQ ID No. 5, 6, 7, 8, 14, 16 or 18.

7. Diagnostic composition containing a nucleotide chip according to Claim 1 or 2, a protein chip according to Claim 3 or an antibody chip according to one of Claims 4 to 6.

8. Method for diagnosing an illness caused by types of the Candida species, wherein a sample to be tested is brought into contact with a nucleotide chip according to Claim 1 or 2, a protein chip according to Claim 3 or an antibody chip according to one of Claims 4 to 6, in a suitable medium, and an interaction between the sample to be tested and at least one of the specified chips is analysed.

9. Method for discovering and identifying substances that are therapeutically effective against illnesses caused by types of the Candida species, wherein a substance to be tested is brought into contact with a nucleotide chip according to Claim 1 or 2, a protein chip according to Claim 3 or an antibody chip according to one of Claims 4 to 6, in a suitable medium, and an interaction between the substance to be tested and at least one of the specified chips is analysed.

## Revendications

1. Puce à nucléotides comprenant un support rigide et au moins une séquence de nucléotides fixée sur celui-ci, et appropriée pour l'identification et la transcription d'un gène codant pour une protéine spécifique à l'hyphe, choisie dans le groupe constitué par :
(a) une séquence de nucléotides, définie dans SEQ ID n° 1, 2, 3, 4, 12, 13, 15 ou 17 ou un brin complémentaire de celle-ci,
(b) une séquence de nucléotides, codant la séquence d'acides aminés, définie dans SEQ ID n° 5, 6, 7, 8, 14, 16 ou 18, ou un brin complémentaire de celle-ci, et
(c) une séquence de nucléotides hybridée avec l'une des séquences de nucléotides (a) ou (b).

2. Puce à nucléotides selon la revendication 1, la séquence de nucléotides étant une séquence d'ADN, d'ARN ou d'APN.

3. Puce à protéines comprenant un support rigide et au moins une protéine spécifique à l'hyphe fixée sur celui-ci, choisie dans le groupe constitué par :
(a) une protéine avec une séquence d'acides aminés, définie dans SEQ ID n° 5, 6, 7, 8, 14, 16 ou 18, et
(b) une protéine avec une séquence d'acides aminés qui présente une identité de séquence d'au moins 80 % par rapport à l'une des séquences d'acides aminés définies dans SEQ ID n° 5, 6, 7, 8, 14, 16 ou 18.

4. Puce à anticorps comprenant un support rigide et au moins un anticorps fixé sur celui-ci qui est dirigé spécifiquement contre une protéine de SEQ ID n° 5, 6, 7, 8, 14, 16 ou 18.

5. Puce à anticorps selon la revendication 4, l'anticorps étant un anticorps monoclonal, polyclonal et/ ou modifié.

6. Puce à anticorps comprenant un support rigide et au moins un anticorps fixé sur celui-ci et dirigé spécifiquement contre un anticorps contre une protéine de SEQ ID n° 5, 6, 7, 8, 14, 16 ou 18.

7. Composition diagnostique contenant une puce à nucléotides selon la revendication 1 ou 2, une puce à protéines selon la revendication 3 ou une puce à anticorps selon l'une des revendications 4 à 6.

8. Procédé pour diagnostiquer une maladie provoquée par des espèces du genre Candida, selon lequel un échantillon à tester dans un milieu approprié est mis en contact avec une puce à nucléotides selon la revendication 1 ou 2, une puce à protéines selon la revendication 3 ou une puce à anticorps selon l'une des revendications 4 à 6, et on établit une interaction entre l'échantillon à tester et au moins l'une des puces mentionnées.

9. Procédé pour détecter et identifier des substances thérapeutiquement actives contre des maladies provoquées par des espèces du genre Candida, selon lequel une substance à tester dans un milieu approprié est mise en contact avec une puce à nucléotides selon la revendication 1, une puce à protéines selon la revendication 3 ou une puce à anticorps selon l'une des revendications 4 à 6, et on établit une interaction entre la substance à tester et au moins l'une des puces mentionnées.
